(19) Europäisches Patentamt / European Patent Office / Office européen des brevets

(11) **EP 2 926 819 B1**

## (12) EUROPEAN PATENT SPECIFICATION

(45) Date of publication and mention
of the grant of the patent:
**28.02.2018 Bulletin 2018/09**

(21) Application number: **13858112.9**

(22) Date of filing: **26.10.2013**

(51) Int Cl.:
*A61K 33/00* $^{(2006.01)}$    *A61P 17/00* $^{(2006.01)}$
*A61P 9/10* $^{(2006.01)}$    *A61P 25/02* $^{(2006.01)}$
*A61P 25/16* $^{(2006.01)}$    *A61P 9/00* $^{(2006.01)}$
*A61P 1/04* $^{(2006.01)}$    *A61P 1/00* $^{(2006.01)}$
*A61P 1/16* $^{(2006.01)}$    *A61P 1/18* $^{(2006.01)}$
*A61P 11/00* $^{(2006.01)}$    *A61P 13/12* $^{(2006.01)}$
*A61P 13/08* $^{(2006.01)}$    *A61P 19/08* $^{(2006.01)}$
*A61P 9/14* $^{(2006.01)}$    *A61P 27/16* $^{(2006.01)}$
*A61P 37/08* $^{(2006.01)}$    *A61P 37/02* $^{(2006.01)}$
*A61P 31/00* $^{(2006.01)}$

(86) International application number:
**PCT/CN2013/086018**

(87) International publication number:
**WO 2014/082514 (05.06.2014 Gazette 2014/23)**

(54) **METHOD FOR INITIATING STEM CELLS OF MAMMALS AND USE OF CHLORINE DIOXIDE IN PREPARATION OF DRUG FOR INITIATING STEM CELLS OF MAMMALS**

VERFAHREN ZUR INITIIERUNG VON SÄUGERSTAMMZELLEN UND VERWENDUNG VON CHLORDIOXID BEI DER HERSTELLUNG EINES ARZNEIMITTELS ZUR INITIIERUNG VON SÄUGERSTAMMZELLEN

MÉTHODE D'INITIATION DE CELLULES SOUCHES DE MAMMIFÈRE ET UTILISATION DU DIOXYDE DE CHLORE DANS LA PRÉPARATION D'UN MÉDICAMENT POUR L'INITIATION DE CELLULES SOUCHES DE MAMMIFÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2012 CN 201210499300**

(43) Date of publication of application:
**07.10.2015 Bulletin 2015/41**

(73) Proprietor: **Liu, Xuewu**
**Beijing 100123 (CN)**

(72) Inventor: **Liu, Xuewu**
**Beijing 100123 (CN)**

(74) Representative: **Glas, Holger**
**Maiwald Patentanwalts GmbH**
**Elisenhof**
**Elisenstrasse 3**
**80335 München (DE)**

(56) References cited:
**WO-A1-89/10747**    **WO-A2-01/27936**
**WO-A2-02/02061**    **WO-A2-2010/111137**
**WO-A2-2011/090932**    **CN-A- 102 441 006**
**CN-A- 103 040 860**

- QU RUO ET AL.: 'Stability of chlorine dioxide treat of severe corneal ulcers efficacy.' CHINESE OURNAL OF CRITICAL CARE MEDICINE. vol. 19, no. 9, 30 September 1999, page 521, XP008179573
- CAI, WEILING ET AL.: 'Observation on effect of patients with nasopharyngeal radioactivity oral cavity mucositis prevented and treated by moerlun gargarism.' CHINESE NURSING RESEARCH vol. 26, no. 3B, 31 March 2012, pages 720 - 721, XP055259872
- XUE, GUANGBO ET AL.: 'Prospect stable cholrine dioxide.' SHANGHAI LABORATORY ANIMAL SCIENCE vol. 14, no. 3, 4, 31 December 1994, pages 133 - 134, XP008179563

**Description**

TECHNICAL FIELD

The present invention belongs to regenerative medicine, and relates to the method of activating stem cells in an animal and the application of chlorine dioxide for preparing the medicine used for activating proliferation, migration and differentiation of stem cells in an animal, for the treatment of an autoimmune disease.

BACKGROUND ART

Diseases occurring in human are classified roughly as organic diseases and functional diseases. In conventional medicine, medical treatment is mainly used for functional diseases, while surgical treatment and medical treatment are used for organic diseases. However, most of medical treatments are symptomatic treatment, which cannot radically cure diseases in the most cases. In addition, surgical treatment is an invasive treatment method, which is practiced by extirpating all or a portion of the injured organ, resulting in partial or complete losses of organ functions. On the other hand, organ transplantation and artificial organs are used as alternative medical methods to surgical treatment. However, a lot of problems exist in organ transplantation, for example, technical problems such as side effects caused by immunosuppressants used to suppress rejection reaction, as well as social problems such as a serious shortage of donors and increased health care costs. In addition, a lot of problems also exist in treatment using artificial organs, which include technical problems such as inability to replace the organic functions and biocompatibility, as well as social problems such as increased health care costs.

Regenerative medicine is currently attracting attention as a new treatment method which solves these problems. Regenerative medicine is termed as a treatment method which aggressively utilizes cells to regenerate construction and restore the function of tissues and organs, for which the functions fall into functional disorder or dysfunction due to illness or accidents. Regenerative medicine is broadly classified into the following three types based on the mode of cell utilization:

(1) a method comprising the steps of collecting embryonic stem cells, placental blood-derived mononuclear cells, blood-derived mononuclear cells or bone marrow-derived mononuclear cells from the donor, inducing the cell proliferation and/or differentiation in vitro culture, and introducing the selected undifferentiated (stem cells and/or progenitor cells) and/or differentiated cells into the patient's body by implantation;

(2) a method comprising the steps of collecting adult stem cells, blood-derived mononuclear cells or bone marrow-derived mononuclear cells from the said patient, inducing the cell proliferation and/or differentiation in vitro culture, and introducing the selected undifferentiated (stem cells and/or progenitor cells) and/or differentiated cells into the patient himself body by implantation; and

(3) a method comprising the step of stimulating the body of the patient (e.g., by administration, physical exercise or physicotherapeutics, etc.) for activating stem cells present in the injured organs or tissues of the patient in situ (the stem cells are undamaged), and/or blood-derived or bone marrow-derived stem cells of the patient, without inducing proliferation and/or differentiation of stem cells in vitro culture. This method is defined as "in situ tissue regeneration".

The current mainstream of regenerative medicine involves methods by which cells are introduced from outside into patient's body by implantation (i.e., the methods of (1) and (2) described above). However, it's not large-scale applied extensively, and the method (1) would involve medical ethics.

Methods in which differentiated cells are introduced from outside of the patient's body are used in the fields of dermatology, opthalmology and orthopedic surgery which targets tissues or organs (e.g., skin, bone, cartilage, cornea and muscle tissues) constructed from one type or extremely limited types of cells. However, with respect to solid organs (e.g., heart, liver, lungs, kidneys and brain) constructed from multiple types of cells, technology for suitably controlling the behavior of these multiple types of differentiated cells has not yet reached the practical level.

On the other hand, the following methods are known for introducing undifferentiated cells from outside of the patient's body.

1) The method of angiogenic therapy involving introduction of stem cells from outside of the patient's body to promote angiogenesis and form new blood vessels for serious ischemic diseases and cardiovascular diseases (Weissman I L: Translating stem and progenitor cell biology to the clinic: Barriers and opportunities. Science 287:1442-1446, 2000).

2) The method of vasculogenesis utilizing introduction of embryonic stem cells (ES cells) from outside of the patient's body (McCloskey K E, et al. Use of embryonic stem cell-derived endothelial cells as a cell source to generate vessel structures in vitro. Tissue Eng 11:497-505, 2005). This method has not reached practical application, since the methods for culturing ES cells, introducing differentiation of the cells and acquiring differentiated cells and so on have not been adequately established, and the methods would involve medical ethics.

3) The method of autologous bone marrow transplantation utilizing bone marrow-derived mononuclear cells isolated from bone marrow of patient himself, and directly introducing the cells into the affected site for formation of new blood vessels (Sato Y, et al. Can a bone marrow cell contribute to organ regeneration? In vivo analysis using transgenic rats with reporter genes. Transplantation Proceedings 37:273-275, 2005). This method has problems such as a small number of stem cells are obtainable, physical burden and risk exists in the patient for collection of a large amount of bone marrow by general anesthesia. Moreover, there is also a considerable difficulty in controlling the differentiation of the implanted cells.

In addition, in regenerative medicine using methods involving the introduction of stem cells from outside of the patient's body, a common problem is the occurrence of complications attributable to excessive regeneration and/or excessive repair by the implanted cells. Certainly, immunological rejection is also one of the major causes of complication.

In contrast, methods utilizing "in situ tissue regeneration" make it possible to recover function by regenerating the injured organs and/or tissues by "activating" stem cells (i.e., resident stem cells, and/or blood-derived or bone marrow-derived stem cells) originally present in inside of the patient's body (the patient is defined as the person who needs some medical method to achieve its goal, such as aesthetic goal rather than the medically diseased patient).

Research from available literature has shown that the stem cells with regenerative ability existed in the liver, nervous system (particularly in the central nervous system such as brain), skin, fatty tissue, retina, cornea, skeletal muscle and even in the heart (Garry D J, et al. Ponce de Leon's fountain: stem cells and the regenerating heart. Am J Med Sci 329(4): 190-201, 2005). Currently, it is considered that resident stem cells are present in all organs and tissues (Weissman I L. Translating stem and progenitor cell biology to the clinic: Barriers and opportunities. Science 287:1442-1446, 2000; Garry D J, et al. Ponce de leon's fountain: stem cells and the regenerating heart. Am J Med Sci 329(4):190-201, 2005). The skilled person in the filed basically can observe that the small wounds of all organs and tissues can heal, from which it can be deduced that the stem cells are distributed widely and evenly in human body and are prepared to repair the wounds around. Of course, with regard to the big wounds (e.g., the shortest wound edge is more than 1cm), the stem cells are unable to repair it completely once and the wound healing only can be replaced by scar (multiple stem cell therapies can repair scar).

Resident stem cells and/or blood-derived or bone marrow-derived stem cells are known from the literature below to be able to differentiate into different types of tissue cells depending on the different inducing microenvironment condition in which they are present, such as different types of contacting cells, contents of extracellular matrix, cytokines and growth factors containing in the inducing microenvironment condition. For example:

(1) when neural stem cells are cultured in the presence of neurotrophic factor or growth factor, they form single cell-derived colonies referred to as neurospheres (i.e., self-replication), and these neurospheres differentiate into neurons, astrocytes and oligodendrocytes based on different inducing microenvironment condition (i.e., multipotency). In addition, when neurosphere was sub-cultured in vitro, another neurosphere can be formed from single neurosphere-derived cell (Reynolds B A and Weiss S. Generation of neurons and astrocytes from isolated cells of the adult mammalian central nervous system. Science 255:1707-1710, 1992);

(2) although neural stem cells only differentiate into gliacytes when neural stem cells obtained from adult rat spinal cord are transplanted into the spinal cord of another adult rat, they differentiate into neurons when transplanted into the hippocampus dentate gyrus (Shihabuddin L S, et al. Adult spinal cord stem cells generate neurons after transplantation in the adult dentate gyrus. The J Neuroscience 20:8727-8735, 2000);

(3) when neural stem cells are cultured with vascular endothelial cells, the proliferation of neural stem cells and differentiation into neurons are promoted (Shen Q, et al. Endothehal cells stimulate self-renewal and expand neurogenesis of neural stem cells. Science 304:1338-1440, 2004).

(4) when bone marrow-derived mesenchymal stem cells (BM-MSCS) are injected to the around of the wound of diabetes rates, bone marrow-derived mesenchymal stem cells (BM-MSCS) can be differentiated into epidermal cells, cytokeratin and gland cell, meanwhile angiogenesis and capillary density are promoted (Yaojiong Wu, et al. Mesenchymal Stem Cells Enhance Wound Healing Through Differentiation and Angiogenesis. STEM CELLS.Volume 25, Issue 10, 2648-2659, October 2007).

On the other hand, for the purpose of regenerative medicine utilizing proliferation, the promotion methods for increasing resident stem cells, and/or blood-derived or bone marrow-derived stem cells are known as follows:

(1) when EGF was administered to animals with a model of cerebral ischemia, neural stem cells proliferation was promoted, and about 20% of the lost cells in infarcted area are regenerated (Teramoto T, et al. EGF amplifies the replacement of parvalbumin-expressing striatal interneurons after ischemia. The J Clinical Investigation 111:1125-1132, 2003);

(2) when the hyperlipemia therapeutic drug, (statin 3-hydroxy-3-methyl-glutaryl coenzyme A (HMG-CoA) reductase inhibitor) was administered to arteriosclerosis patients, the levels of bone marrow-derived hemangioblasts or endothelial progenitor cells increased in the blood (Walter D H, et al. Statin therapy accelerates reendothelialization: A novel effect involving mobilization and incorporation of bone marrow-derived endothelial progenitor cells. Circulation 105:3017-3024, 2002);

(3) in addition to the above-mentioned EGF, numerous growth factors such as VEGF, FGF (b-FGF), PDGF, NGF and HGF, cytokines such as G-CSF, GM-CSF, erythropoietin (EPO), and other bioactive substance such as estrogen and lipids, etc. are reported useful for increasing stem cells (Takeyama K, Ohto H: PBSC mobilization. Transfus Apher Sci 31:233-243, 2004; Aicher A, Zeiher A M, Dimmeler S: Mobilizing endothelial progenitor cells. Hypertension 45:321-325, 2005). However, there are only two or three of the above-mentioned substances which have been developed into pharmaceuticals such as G-CSF and b-FGF. Moreover, G-CSF has the risk of cancerogenesis, while b-FGF has the risk of side effects such as vascular occlusion during intravenous injection. In addition, in consideration of the diversity of side effects attributable to growth factors targeting numerous types of cells, adequate clinical efficacy has yet to be obtained in clinical studies for VEGF and b-FGF, which were developed as growth factors having a small number of target cell types. Moreover, EPO has side effects including elevation of blood pressure. Chinese patent Successive Administration Regimen of Neural Stem Cells Proliferation Reagent and Neural Stem Cells Differentiation Reagent of publication number CN101405021A mentioned the methods of successive administration in low dose of neural stem cells proliferation reagent such as hCG (human chorionic gonadotrophin), prolactin and EPO to animal subject in days;

(4) early study showed that soluble protein factor, which can inhibit or stimulate stem cell proliferation, existed in normal and regenerative extracts from bone marrow (summarized by: Lord and Wright, Blood Cells. 6:581-593,1980; Wright and Lorimore, Cell Tissue Kinet. 20:191-203, 1987; Marshal land Lord, Int Rev. Cyt. 167:185-261,1996). However, the stem cell irritant has not been purified from the prepared extracts from bone marrow mentioned by Lord et al;

(5) thrombin-like enzyme (preferably batroxobin) as the activator of stem cell and/or progenitor cell was mentioned at Chinese patent *Activator of Stem Cell and/or Progenitor Cell* of publication number CN101278045A. It is learned from the implementations that batroxobin played a activating role in the stem cells in in-situ tissue. However, the invention just opened its activating role in cells because of lacking theoretical foundation and broad clinical data.

Apparently, clinical medicine requires the rational and convenient utilization of stem cells inherent in body and spread all over organs or tissues by way of in situ tissue regeneration, in which the mentioned substance can activate stem cell proliferation, migration and differentiation with few or no side effects and come into play rapidly and moderately according to the state of advancement and the degree of injured organs and/or tissues to which regenerative medicine is applied.

It's easy for the skilled person in the field to accept the leading role of stem cells in tissue. Besides, the tissue regeneration led by stem cells also has immunoregulatory effect, from which the skilled person in the filed deduced that stem cell can treat hypersensitivity (including allergic diseases and autoimmune diseases).

Pathogenesis of hypersensitivity disease is complicated, which often shows mixed type with a certain type based in clinical practice. For example, type I and III can appear simultaneously, so can type II and type III as well as type II, III and IV.

Hypersensitivity disease means allergic reaction when exogenous antigens (such as bacteria, virus, pollen, dust, etc.) are read as harmful objects. In this condition, phagocytes in immune cells would be activated and release histamine and prostaglandin, resulting in telangiectasia, increase of vascular permeability, itching, smooth muscle contraction, reflecting action, etc. Mildly, erythema and measle would appear. Badly, inflammation such as swelling and fever would appear. Allergy refers to the abnormal reactions, also called type I allergy. It does not lead to tissue or organ damage in general, while some type I anaphylactic reactions turn into chronic symptoms(desensitization treatment is useless) or lead to tissue and organ damage and difficult to heal when accompanied by other type anaphylactic reactions. At present, glucocorticoid and immunosuppressor are the main medicines utilized in clinical practice. More effective tissue regeneration therapy is required if the anaphylactic reaction turns into chronic symptom since the general treatment is not effective enough.

Most autoimmune disease tissue damages are caused by type II, III, IV allergic reactions: function of autoantibody; function of immune complex; function of T cells; function of macrophage and natural killer cell.

Common features of autoimmune diseases: the quantity and severity of T cells which has autoantibody and/or autoreactivity are parallel; the involvement is mainly the distribution part of autoantigens aimed by autoantibody or sensitized lymphocyte; movable and transferable; most autoimmune diseases are agnogenic; delay with alternates seizure and remission in disease course; female patients are common, incidence increases with age, it has genetic tendency; disease has overlapping phenomenon; immunosuppressor has a certain effect.

Autoimmune diseases refer to any disease caused by misrouting body healthy cells and/or tissue immune cells. Autoimmune diseases are featured by the damage and/or dysfunction of organ, tissue or histologic type(such as skin, pancreas, brain, thyroid or gastrointestinal tract) abnormal targeted self proteins, self polypeptide, self peptide and/or other self molecular T and B lymphocyte, leading to clinical manifestation of the disease(Marrack et al., Nat Med 7, 899-905,2001). Autoimmune diseases include the diseases (organ specific) affecting specific tissue and/or organ and the diseases (non-organ specific diseases) affecting multiple tissues and/or organs. For some diseases, it may partly depend on whether autoimmune response targets antigen of particular organ or antigen spreading all over body. The surface feature of tissue specific autoimmunity is selectively targeting single organ or single cell type. For example, autoimmune response targets widely existing protein-tRNA synthetase histidyl in polymyositis while sarcous autoimmune destruction is the main involved clinical manifestation.

Body immune system is able to recognize self and non-self antigen substance. Under normal circumstances, immune system does not produce immune response to autologous tissue antigen or only produce the slightest immune response, which is also called self-tolerance. Self-tolerance is maintained by immune system through active adjustment of multiple mechanisms so as to prevent autologous histiocyte from damage caused by the attack made by immune response. In some cases, self tolerance is damaged and immune system produces obvious immunoresponse to autologous tissue, namely antibody aimed at autologous tissue or sensitized lymphocyte in body, which is called autoimmunity. The damaged mechanism of self tolerance is not clear, but immunity problems of body with inherited tendency caused by some exogenous materials such as virus, bacterial, toxin and some chemical substances play main role. Autoimmunity is physiological in many cases, because autoimmune response within limits is helpful to remove senile and aberrant autologous cells, and can adjust immunoresponse. Autoimmune response autologous would cause tissue damage and corresponding dysfunction, resulting in autoimmune diseases only when autoimmune response surpasses physiological limit or persists too long.

Currently, the common therapies of autoimmunity are to utilize glucocorticoid and immunosuppressive drug to relieve inflammatory response caused by the affect on immune system and to inhibit immune system. However, the therapies would cause serious adverse reactions such as serious infection, myelosuppression, etc. And they can only slow progress of the disease instead of effecting a radical cure.

Since autologous bone marrow transplantation was reported to treat systemic lupus erythematosus successfully by Marmont in Italy in 1996, many countries around the world started to study the application of stem cells on autoimmunity disease. For example, placenta stem cells are used to treat autoimmunity disease (Chinese patent with publication number CN101657206A); body fat-derived stem cells are used to type I diabetes (Vikash Chandra, et al. Islet-Like Cell Aggregates Generated from Human Adipose Tissue Derived Stem Cells Ameliorate Experimental Diabetes in Mice.PLoS One. 2011;6(6):e20615. Epub 2011 Jun 7.); neural progenitor cells are used to treat immunologic disease(Wei Cao, et al. Leukemia Inhibitory Factor Inhibits T Helper 17 Cell Differentiation and Confers Treatment Effects of Neural Progenitor Cell Therapy in Autoimmune Disease, Immunity, Volume 35, Issue 2, 273-284, 11 August 2011); insulin-secretion cells generated by enteric progenitor cells in intestinal tract are used to treat type I diabetes(Chutima Talchai, et al. Generation of functional insulin-producing cells in the gut by Foxo1 ablation. Nature Genetics.44,406-412(2012)); umbilical cord blood stem cells are used to treat type I diabetes (Yong Zhao, Zhaoshun Jiang, Tingbao Zhao, Mingliang Ye, Chengjin Hu, Zhaohui Yin, et al. "Reversal of type 1 diabetes via islet beta cell regeneration following immune modulation by cord blood-derived multipotent stem cells." BMC Medicine 2012, 10:3, 10 January 2012.); bone marrow mesenchym stem cells by intravenous are used to treat autoimmune myasthenia myasthenia gravis(Yu, J, et al. Intravenous Administration of Bone Marrow Mesenchymal Stem Cells Benefits Experimental Autoimmune Myasthenia Gravis Mice Through an ImmunomodulatoryAction, Scandinavian Journal of Immunology, 2010; 72 (3): 242); hematogenous stem cells are used to treat multiple sclerosis of old animal(Julia M. Ruckh, et al. Rejuvenation of Regeneration in the Aging Central Nervous System, Cell Stem Cell, Volume 10, Issue 1, 96-103, 6 January 2012); mesenchymal stem cells are used to treat systemic sclerosis and immunal regulation of stem cells are discussed(Kentaro Akiyama, et al. Mesenchymal-Stem-Cell-Induced Immunoregulation Involves FAS-Ligand-/FAS-Mediated T Cell Apoptosis. Cell Stem Cell. Volume 10, Issue 5, 544-444, 26 April 2012).

The two functions of stem cells mainly used in autoimmune disease treatment utilizing stem cells include immunomodulatory and tissue regeneration. In terms of immunomodulatory of stem cells, some study reported that mesenchymal stem cells had clinical effect of inhibiting proliferation of T cells and B cells to regulate body autoimmune response, while the immunomodulatory is different from unimolecular regulation of immunosuppressive agents common in clinic. MSCs achieves it by means of multimolecular participation, multipath regulation and paracrine and its immunomodulatory interacts with microenvironment of systemic inflammatory(Pittenger, M.F., Martin, B.J. Mesenchymal stem cells and their potential as cardiac therapeutics. Circ. Res., 2004, 95, 9-20). In addition, tissue regeneration initiated by stem cells have native immunoregulation capability(because new tissues are recognized as "self" by body immune system).

Of course, the treatment of autoimmune diseases utilizing stem cells is still by stem cell transplantation rather than the use of stem cells in in-situ tissue.

Being an internationally accepted new efficient, broad-spectrum and safe fungicide and preservative, chlorine dioxide is an ideal substitute of hypochlorites and has been applied widely. Related organizations in America, Western Europe, Canada and Japan such as EPA, FDA and USDA approved and recommended chlorine dioxide to be used for food, food processing, pharmacy, hospital, disinfection of public environment, mould proof, preservative and fresh-keeping of food and so on. Chlorine dioxide has been listed as a safe and efficient disinfectant with A1 level by WHO and FAO. In order to control the generation of carcinogenic, teratogenic and mutagenic substances, Euramerican developed countries have widely used chlorine dioxide to replace chlorine for disinfection of drinking water. However, chlorine dioxide has not yet been accepted by market as a medicine. Some patents involves in some treatments of diseases utilizing chlorine dioxide (such as CN102137651A, CN101641104A and CN1199633C), these patents still only utilize strong oxidizability and ability of anti-infection which can kill skin pathogenic microorganism of chlorine dioxide. Patent of publication number US5750108 mentioned that chlorine dioxide can stimulate hair follicle to grow new hair, but the maximum capacity of chlorine dioxide was not given full play because of low concentration, so the effect was not obvious; patent of publication number CN102441006A provided a external pilatory containing chlorine dioxide which can stimulate hair growth in alopecia area in short time and the recoverable cases, but the patent has not found the potential mechanism of action of activating stem cells of chlorine dioxide.

Furthermore, international application WO 89/10747 A1 refers to a method for treating dermatologic diseases caused by microbial overgrowth or inflammation such as psoriasis, fungus infections, eczema, dandruff, acne, herpes lesions by genital and leg ulcers. There is further described a lubricating anti-viral composition that is effective in preventing the transmission of the HIV virus and other sexually transmitted diseases. There is also disclosed systematic anti-inflammatory compositions and formulations and a method of reducing tissue inflammation in tissues such as bowel, muscle, bone, tendon and joints. International application WO 2011/090932 A2 relates to a method for treating acute and chronic sinusitis, and in particular, severe chronic sinusitis by exposing affected tissue of the sinus and contiguous tissue in the, nasal cavity and greater oral cavity to effective amounts of chlorine dioxide as a bioactive agent. International application WO 2010/111137 A2 refers to a composition and method for suppressing or preventing fibrous adhesion formation using a multicomponent aqueous oxychlorine composition. Fibrous adhesions typically form during healing of tissue, for example following a surgical procedure. A multicomponent oxychlorine composition is provided for irrigating the tissue which minimizes post-surgical adhesion formation, the composition containing both chlorine dioxide and chlorite ion, and a complex ion thereof.

DISCLOSURE OF INVENTION

The inventive subject-matter of the present application is defined in claim 1 and in the subsequent dependent claims.

A purpose of the present invention is to provide a method of activating proliferation, migration and differentiation of stem cells in animal on the basis of current technical research. The method can rapidly and moderately activate proliferation, migration and differentiation of stem cells with few or no side effect and can persistently give play to effect of activating proliferation, migration and differentiation, in the treatment of autoimmune diseases. In order to achieve the aims, the technical plan of the present invention is a method of activating proliferation, migration and differentiation of stem cells, including preparation of activating agent of stem cells containing chlorine dioxide and procedure of giving said activating agent to target tissues of animal, wherein said activating agent provides effective dose of chlorine dioxide when it is given to to target tissues of animal.

Further, the method of preparing activating agent of stem cells containing chlorine dioxide is to dissolve chlorine dioxide gas in acid solution A containing acid pH modifier with pH value of 1.5 ~ 6.5 to prepare chlorine dioxide solution of 500 ~ 2900ppm.

Further, the method of preparing activating agent of stem cells containing chlorine dioxide is that aqueous solution of 1%~40% is prepared by dissolving chlorine dioxide precursor in water, acid solution B containing acid pH modifier is added so as to adjust pH value of the mixed solution to 1.5~6.5.

Further, the method of preparing activating agent of stem cells containing chlorine dioxide is to dissolve chlorine dioxide precursor in water to prepare aqueous solution C of 1% ~ 40%; dissolve acid pH modifier in water to prepare acid solution D; mix solution C and D and adjust pH value of the mixed solution to 1.5 ~ 6.5 before use.

Further, chlorine dioxide precursor refers to sodium chlorite, potassium chlorite, lithiumchlorit, calcium chlorite, magnesium chlorite and barium chlorite.

Further, acid pH modifier refers to citric acid, acetic acid or sodium dihydrogen phosphate.

Further, activating agent of stem cells is directly given to injured organs or tissues by arterial injection, intramuscular injection, subcutaneous injection, intracardiac injection, intrathecal injection, intra-articular injection, puncture injection, rectal administration, nasal delivery, transdermal delivery or inhalation delivery.

Further, activating agent of stem cells is produced to injection, ointment, inhalant, collunarium, lotion, suppository, patch, cataplasm, tablet, oral liquid, capsule, granule, electuary, pill or syrup.

The present invention provides the application of drug preparation of activating proliferation, migration and differentiation of chlorine dioxide, wherein the drug is used for the treatment utilizing regenerative medicine by activating proliferation, migration and differentiation of stem cells. The treatments utilizing capability of tissue regeneration and immunoregulation of stem cells refer to the treatments of an autoimmune disease.

BEST MODE FOR CARRYING OUT THE INVENTION

The following provides a detailed explanation of the present invention.

The mechanism provided by the invention is described before the mode for carrying out the invention.

An American clinical trial report No.NCT01341041 *Chlorine Dioxide Versus Saline for Wound Irrigation* (by Rhode Island Hospital) showed that chlorine dioxide has the following effects compared with normal saline: promote healing and reduce scar formation. In animal test. It is reported that chlorine dioxide has the effect of speeding haemostasis and promoting healing on wound (Jin Wei et al., the trial study on promotion of chlorine dioxide on wound healing of rabbit, Shanxi Medical University Journal, Issue 7, Volume 42, 2011).

Therefore, it is certain that wound healing is activated by stem cells. Any wound of the tissue will not heal without stem cells. Normally, wound of human tissue can heal normally without any stimulation of medicine. The primary cause lies in the stem cells around that will grow complete new tissue by proliferation, migration and differentiation. The shortage of quantity or differentiation capacity of stem cells will result in scar formation during the healing process or difficult healing in long time. For example, first degree burns can heal naturally without scar; second degree burns can heal; third degree burns can't heal naturally.

Adult stem cells are regard as the source of new tissue in wound healing, including wound healing of skin, liver regeneration, lung regeneration, renal function repair, corneal epithelial healing, nerve regeneration, cardiomyopathy and ischemic cerebral injury are achieved by stem cells proliferation, migration and differentiation in in situ tissue. Mesenchymal stem cells have been proved to participate in each stages of wound healing (Katherine Lau, et al. xploring the role of stem cells in cutaneous wound healing. Experimental Dermatology. Volume 18, Issue 11, Pages 921-933, November 2009).

The inventor also finds that chlorine dioxide has the ability to accelerate healing on skin wound with few scars, such as the wound healing of larger inflammatory wound and diabetic foot.

Adaptive pain will appear after the injury of human tissues, including nociceptive pain and inflammatory pain. Nociceptive pain can prevent body from more serious injury from the outside, so as to protect body (Clifford J. Woolf. What is this thing called pain? J Clin Invest. 2010 November 1; 120(11): 3742-3744); Inflammatory pain can promote the healing of injured tissue(Clifford J. Wool. Pain: Moving from Symptom Control toward Mechanism-Specific Pharmacologic Management. Annals of Internal Medicine (2004), Volume: 140, Issue: 6,Pages: 441-451). Therefore, pain is good for repairing injured tissues and promotes stem cells differentiation to treat tissue injury in regeneration way by stimulating factors.

The inventor also finds that chlorine dioxide solution can produce micro-wound wound and cause short-time pain when it applied on organs and tissues. The inventor believes that the micro-wound has effect of debridement to removal necrosis tissue on one hand; on the other hand, it can guide stem cells to affect tissue regeneration, which is stimulated by pain.

When observing regenerative process of follicle cells, it's found that skin wound healing differentiates with an earlier mode accompanied by stem cells to some extent. For example, regenerative tissues are younger.(Katherine Lau, et al. Exploring the role of stem cells in cutaneous wound healing. Experimental Dermatology. Volume 18, Issue 11, pages 921-933, November 2009). In the study on mouse, healing process of anthropogenic wound can produce new follicle stem cells community and new hair. Meanwhile, both follicle stem cells and epidermal stem cells take part in the cell(including follicle cells and sebocyte) formation in healing process, including stem cell proliferation, migration and differentiation in which Wnt signal plays the main role (Ito M, et al. Wnt-dependent de novo hair follicle regeneration in adult mouse skin after wounding. Nature. 2007 May 17;447(7142):316-20).

The inventor finds that the following phenomenons appear when chlorine dioxide solution is applicable to alopecia area: when chlorine dioxide solution is applied every day, wound-shaped erythema appears at the area applied on chlorine dioxide solution and new hair grow in lopecia area with hair on the third to the fifth day, and new hair grow in lopecia area without hair in about 20 days, which can only be explained by regeneration tissue capacity of stem cells.

Wound of fetus heals rapidly without scar while it's difficult for wound healing of adult with their ages increase, which may present the change of functional status of stem cells(Cecilia Roh, et al. Cutaneous Stem Cells and Wound Healing. Pediatric Research (2006). 59, 100R-103R). It also explained that there is senile possibility in regenerative capacity of stem cells. "Niche" of stem cells is vital to stem cells loss caused by senility. Stem cells locate in niche of other cells and it is well known that niche is vital to function of stem cells. In the study on molecular mechanism of self-renewal of germ stem cells of fruit fly, research personnel finds that cells produce a mrna molecule let-7 with negative regulation effect in RNA along with the change of niche of stem cells, and the increase of let-7 expression will reduce the quantity of active stem cells of niche(Hila Toledano, et al. The let-7-Imp axis regulates ageing of the Drosophila testis stem-cell niche. Nature (2012). Published online 23 May 2012). It follows that senility of regenerative capacity of stem cells embody in the reduced quantity of stem cells to some extent, and signal transmitted by niche plays a vital role.

The inventor also finds that for the same wound (with same area and same depth) treated by chlorine dioxide solution, renewability of people over the age of 60 is lower than that of people under the age of 30, which indicates that the effect of chlorine dioxide solution on stem cells is pretty much exactly the same as prediction result from above theory.

According to above theory and observation of the practice, the inventor finds that chlorine dioxide solution obviously promotes tissue repair from the perspective of wound healing and it can be seen that renascent tissues are more younger.

Redox potential of chlorine dioxide is 1.5 when getting 5 electrons, which is more than oxygen of 1.2. It belongs to strong oxidant because of strong ability to get electrons. The inventor believes that chlorine dioxide is able to promote stem cells proliferation, migration and differentiation of animals: chlorine dioxide destructs common cells by relying on strong oxidation , causing wound on tissues and pain or removing slough, in which body will automatically activate stem cells to heal the wound; chlorine dioxide is a water soluble gas and can be regarded as signal molecule like nitric oxide and hydrogen sulfide, which develops the special ability of signal molecule to activate stem cells to proliferation, migration and differentiation so as to achieve tissue regeneration when entering permeating into body tissues.

Major studies support the theory that nitric oxide can promote wound healing(Majida Rizk, et al. Nitric Oxide and Wound Healing. World Journal of Surgery. Volume 28, Number3 (2004), 301-306,), which especially similar to the chronic wound healing of diaberic(M. B. Witte, et al. Nitric oxide enhances experimental wound healing in diabetes. British Journal of Surgery. Volume 89, Issue 12, Pages 1594-1601, December 2002). Redox potential of nitric oxide is 1.6 when getting 2 electrons in acid condition, which is close to chlorine dioxide, and both of them are water-soluble(the former is slightly water-soluble). The inventor supposes that chlorine dioxide can act on body as ectogenic signal molecule, which differs from endogenic signal molecule such as nitric oxide, and can be regarded as activating agent of stem cells in in situ tissue regeneration.

The inventor supposes that the mechanism of preparation containing chlorine dioxide used suitably for demic in situ tissue regeneration includes the followings:

Firstly, preparation containing chlorine dioxide in the invention can remove necrotic part of injured tissues, providing space for stem cells to activate and achieve tissue repair, meanwhile, the broad space and tissue-specific suggestion stimulate body to repair injured tissue automatically. In addition, as Chinese patent NO. CN101641104A Curative of Infectious Skin and Mucosa Diseases which utilizes the effect of chlorine dioxide of killing broad-spectrum nosopoietic microorganism to treatment of skin disease caused by pathogenic microorganism, chlorine dioxide preparation in the invention can efficiently kill pathogenic microorganism and has the effect of debridement, preventing infection, removing exogenous material (exactly is and/or regarded by immune system) such as allergens.

Secondly, preparation containing chlorine dioxide can produce evenly distributed micro wounds in injured tissues and cause pain to stimulate body to activate factors and cells to repair wound in early mode, making the repaired tissues new and younger, including new tissues recognized by immune system as "self" which have immunoregulation capability.

Thirdly, as ectogenic gas signal molecule, chlorine dioxide in the preparation containing chlorine dioxide increases activity of stem cells and promotes repair of injured tissues by activating stem cells proliferation, migration and differentiation. In addition to activating stem cells to initiate tissue regeneration, chlorine dioxide has the effect of directly or indirectly immunoregulation by stem cells as gas signal molecule, in which the effect played by chlorine dioxide preparation is complicated and comprehensive.

The present invention is based on the above research results, so preparation containing chlorine dioxide as activating agent of stem cells in the invention not only can treat the disease by utilizing stem cells to initiate tissue regeneration, but also can treat the disease by utilizing accompanying immunoregulation capability during tissue regeneration of stem cells as well as achieve debridement and anti-infection of killing pathogenic microorganism by utilizing chlorine dioxide.

Academic research cleared that chlorine dioxide can be used for body safely. Shi Laishun and Xie Chaoren(*Test Observation of Acute Toxicity and Irritation of Stable Chlorine Dioxide*) concluded from tests that stable chlorine dioxide is an innoxious substance for mouse per os of LD50 > 10000mg/kg. The content of chlorine dioxide, is 9.7 ~ 11.4mg/L, whose stimulation score is 0 to skin and eye mucous membrane 48h, so chlorine dioxide belongs to nonirritant substance. Huang Junli et al. concluded from tests that $ClO_2$ aqueous solution of 276.5mg/L, $NaClO_2$ and $NaClO_3$ aqueous solution of 200mg/L and $ClO_2$ mixed solution of total concentration of 553mg/L are actually non-toxic aqueous solution; aqueous solution of $ClO_2$ of 276.5mg/L, $NaClO_2$ of 200mg/L and $NaClO_3$ are non-obvious accumulative toxic aqueous solution; $ClO_2$ mixed solution has no significant influence on rat weight gain and food digestibility($p<0.05$); hemological indexes of rats such as white blood cell count and hemoglobin are not influenced($p<0.05$); the examination on ALT, TP, ALB and GLO showed that control group and female and male of high dose group had no significant difference($p<0.05$); he variance analysis showed that hepatosmatic index(%) and renal index(%) of experimental group and control group had no significant difference($p<0.05$) ; the histopathological results showed that liver tissue and renal tissue of each experimental group and control group had no lesion.

Although the definition of the stem cells targeted by the present invention has not been completely standardized in the relevant technical field, they are defined in the following manner in the present specification on the basis of the concept that a consensus has been obtained.

Stem cells are undifferentiated cells with the self-replication ability and multipotency. Specific examples of stem cells include embryonic stem cells (ES cells), embryonic germ cells (EG cells), somatic stem cells (adult stem cells), hemangioblasts, neural stem cells, hematopoietic stem cells, mesenchymal stem cells and stem cells of other cells (including osteocyte, chondrocyte, myocyte, cardiac myocyte, neuron, tendon cell, adipocyte, pancreocyte, hepatocyte, nephrocyte and follicle cells and so on).

The differentiation and activation stage of activating agent of stem cells in the invention include the whole process from decurrent differentiation of stem cells to terminal stage differentiated cells, including the process in which stem cells differentiate into progenitor cells(progenitor cells are defined as daughter cells of stem cells, their differentiation potential or self-replication ability or both are more restricted), the whole process in which progenitor cells differentiate into differentiated cells and then into terminal stage differentiated cells or some stage of them. The whole stage represents a general mode for stem cells to repair injured tissues. Stem cells in body lead the function of maintenance and repair to tissues. Their operating mode during life cycle can be divided into proliferation, migration and differentiation to achieve the functions. In order to maintain the normal tissue function of body, the modes of self-renewal and cloning behavior of stem cells show accordingly: symmetric cell division by which stem cells achieve proliferation; symmetric cell division and migration of stem cells in neighbouring tissues; asymmetric cell division by which stem cells achieve self-renewal and differentiate into daughter cells(Allon M. Klei, et al.Universal patterns of stem cell fate in cycling adult tissues, Development, August 1, 2011, 138, 3103-3111). In activating agent of stem cells containing chlorine dioxide in this invention, the "activate" includes the whole process of a set of tissue maintenance and repair including activating stem cells proliferation, migration and differentiation. A study showed that differentiated cells or tissue-specific stem cells can change its phenotype and emerge functional characteristics of other histocyte, which is called transdifferentiation. In the invention, transdifferentiation of stem cells which is defined as tissue-specific stem cells turned into cells of other ancestry. If some tissue-specific stem cell lost the ability to regenerate the tissue, transdifferentiation of adjacent stem cells is required when regeneration of the tissue is needed. A small number of transdifferentiation of stem cells exists in body tissues and the invention includes the definition of transdifferentiation into definition of stem cell differentiation.

Stem cells present in adults body can be divided into resident cells, blood-derived cells and bone narrow-derived cells based on their origin and location.

Resident stem cells are defined as cells with self-replication ability and multipotency that are originally present in a specific organ and/or tissue, which contribute to regeneration of that organ and/or tissue in the case said organ and/or tissue is subjected to injury. Specific examples of these resident stem cells include corneal epithelial cells, cardiac stem cells, neural stem cells, vascular EPCs and so on. According to the above description, skilled person in the field can learn that resident stem cells which can be used for in situ tissue regeneration spreads broadly in almost all organs and tissues of human body.

Blood-derived stem cells are defined as cells with self-replication ability and multipotency that are present in the circulating blood, which contribute to regeneration of an organ and/or tissue by migrating and accumulating into that organ and/or tissue in the case said organ and/or tissue is subjected to injury. The blood in this case includes peripheral blood, placental blood, arterial blood, venous blood, blood sampled from the heart, and blood sampled from the ocular fundus. Specific examples of blood-derived stem cells include vascular EPCs, mesenchymal stem cells and so on.

Bone marrow-derived stem cells are defined as cells with self-replication ability and multipotency that are originally present in bone marrow, which contribute to regeneration of an organ and/or tissue by migrating through the blood circulation and accumulating into said organ and/or tissue in the case said organ and/or tissue has been subjected to injury. Specific examples of bone marrow-derived stem cells include vascular EPCs, mesenchymal stem cells and so on.

The activating stem cell in the present invention is able to is able to activate proliferation, migration and differentiation of each of these resident cells, blood-derived cells and bone marrow-derived cells.

In addition, stem cells can be divided into activated-state cells (state in which the cells are proliferating, differentiating or migrating) and inactive-state cells (state in which cells are not proliferating, differentiating or migrating). But the activating agent of the present invention is able to activate stem cells in both activated-state and inactive-state.

The activating agent of the present invention activates stem cells to repair injured tissue and realize generation. Here, "activate" is defined as the three actions indicated below.

(1) Initiation or activation of proliferation of stem cells.
(2) Initiation or activation of migration of stem cells. Migration is defined as stem cells being mobilized from bone marrow, organ and/or tissue into the circulating blood; accumulated in the injured organ and/or tissue from the circulating blood; or moved inside of the organ and/or tissue.
(3) Initiation or activation of differentiation of stem cells.

The activating agent of the present invention can solely contain single preparation of chlorine dioxide or contain some shares of chlorine dioxide precursor. The method of preparing single preparation solely containing chlorine dioxide is as follows:

Method 1: add PH modifier to water to make acid aqueous solution with PH valued of 1.5 ~ 6.5. Chlorine dioxide gas is made by the preparation method (preferable to the response of chlorite and acid) with chlorine dioxide concentration of over 99.9% in normative way. Chlorine dioxide gas is bubbled and dissolved in the acid aqueous solution mentioned above to prepare chlorine dioxide solution of 500 ~ 29000ppm. The solution should be preserved in dark and airtight condition with low premature of 4°C ~ 15°C before use. The aqueous solution in the method is replaceable but chlorine dioxide must be the major substance playing a role in targeted tissues in the method.
Method 2: dissolve chlorine dioxide precursor in water to prepare aqueous solution with concentration of 1% ~ 40% and add acid solution containing PH modifier (preferable to citrate solution with concentration of 2% ~ 50%) to adjust PH value of the mixed solution to 1.5 ~ 6.5. The solution should be preserved in dark and airtight condition with low premature of 4°C ~ 15°C before use. The aqueous solution in the method is replaceable but chlorine dioxide must be the major substance playing a role in targeted tissues in the method.

The method of preparing some shares containing chlorine dioxide precursor is as follows: dissolve chlorine dioxide precursor in water to prepare aqueous solution with concentration of 1% ~ 40%, which is the first solution; use PH modifier to prepare acid solution(preferable to citrate solution with concentration of 2% ~ 50%), which is the second solution. Mix the solutions mentioned above at the scene and adjust its PH value to 1.5 ~ 6.5 before the treatments to diseases listed in the invention, and then apply the solution to the targeted tissues after chlorine dioxide is generated in the response. The aqueous solution in the method is replaceable but chlorine dioxide must be the major substance playing a role in targeted tissues in the method.
Examples of chlorine dioxide precursor can be used in the present invention include alkali chlorite metal salt, alkali chlorite metal salt of soil type. Alkali chlorite metal salt includes sodium hypochlorite, potassium chlorite and lithiumchlorit; alkali chlorite metal salt of soil type includes calcium chlorite, magnesium chlorite, and barium chlorite.
From the perspectives of easy accessibility and excellent persistent of chlorine dioxide activity, sodium chlorite and potassium chlorite are preferable, especially sodium chlorite.
Any acid with cushioning can be used as the PH modifier used in the present invention.
Examples of organic acid or its salt include formic acid, acetic acid, propionic acid, butyric acid, lactic acid, pyruvic acid, citric acid, malic acid, tartaric acid, gluconic acid, glycolic acid, fumaric acid, malonic acid, maleic acid, oxalic acid, succinic acid, crylic acid, crotonic acid, glutaric acid and their salt.
Examples of inorganic acid include phosphonium, boronic acid, metaphosphoric acid, pyrophosphoric acid and sulfamic acid. Examples of inorganic acid salt includedihydric phosphate (sodium salt and leopoldite, the same below), mixture of dihydric phosphate and hydrophosphate. PH modifier can be used alone or simultaneously with two or more kinds.
From the perspective of physical safety, citric acid, acetic acid and sodium dihydrogen phosphate are preferable PH modifier.
In addition, the final preferable PH value of chlorine dioxide liquor is 1.5 ~ 5.5, more preferable PH value is 1.5 ~ 3.5.
The activating agent containing chlorine dioxide of the present invention is preferable to liquor.
The dosage of the activating agent of the present invention varies from the age, body weight, disease character and disease status of patient. For adult, the daily dosage of chlorine dioxide is 1mg to 5000mg, and the daily dosage of 1mg to 1000mg is preferable.
The administration routes of the activating agent of the present invention are multifarious. It can be delivered systemically including intravenous, intra-arterial or intraperitoneal administration; it also can be directly delivered to affected part by cutaneous penetration and puncture.
The activating agent of the present invention can be delivered by any way which is able to reach the expected tissues, such as intravenously guttae, intravenous, arterial injection, intramuscular injection, subcutaneous injection, intradermal injection, intracardiac injection, intraperitoneal injection, intrathecal injection, intra-articular injection, puncture injection, rectal administration, sublingual administration, nasal delivery, transdermal delivery, inhalation or local drug delivery to injured organs or tissues and so on.
The effective dose of the activating agent is 0.1 ~ 500mg/kg of chlorine dioxide daily because of the size of targeted tissue. Sometimes the targeted tissue is easier to be expressed by area, so the effective range of the activating agent is 0.1 ~ 500mg/100cm$^2$ of chlorine dioxide. The efficacy comes into play at least after 10 days in the mentioned dose.

In any case, the activating agent only comes into play without the help of other supplementary material when the effective dose of chlorine dioxide is applied to the target in appropriate way. There is no limit on the way and procedure.

Any preparation in *Preparations Rules of Chinese Pharmacopoeia* can be used as the dosage form of the activating agent in the present invention. Examples of the dosage form of the activating agent include injection directly applied to body (such as suspension and emulsion); ointment (such as grease ointment, emulsion ointment and water-soluble ointment); or tablet (such as icing, film and glaze), liquid preparations, capsule, granule, powder, pill, syrup, lozenge and so on. The preparations can be prepared according to *Preparations Rules of Chinese Pharmacopoeia.*

In addition, the administration of the activating agent in the present invention also can include solid or liquid carrier or interventional therapy material. Examples of solid or liquid carrier include solvent, stabilizer, cosolvent, emulgator, clouding agent, buffering agent, isoosmotic agent, colorant, matrix, thickener, excipient, lubricant, binder, disintegrant, coating agent, corrigent, conditioner, foaming agent, super absorbent resin, surfactant, permeation enhancer, PH modifier and so on.

The examples include deionized water, lactose, white sugar, fructose, glucose, mannose, sorbitol or sugar alcohol; cellulose and its derivative such as crystalline cellulose, methyl cellulose, ethocel, hydroxy propyl cellulose, low-substituted hydroxypropyl cellulose, hydroxypropyl methyl cellulose, hydroxypropyl methylcellulose phthalate, hydroxypropyl methylcellulose acetate succinate, carboxy methyl cellulose, calcium cellulose lycolate, carboxymethyl cellulose sodium, cross-linked sodium carboxymethyl cellulose, hydroxy methyl ethyl cellulose, cellulose acetate phthalate; corn starch, wheat starch, rice starch, starch potatoe, cyclodextrin, amylopectin and so on; Heat polymer,(seaweeds, vegetable uncilage and protein) agar, sodium alginate, Arabic gum, gelatin, collagen, shellac, yellow starch glue, xanthan gum; synthetic macromolecule such as polyvinyl pyrrolidone, Amino alkyl methacrylate copolymer,methacrylic acid copolymer, hydroxy vinyl copolymer, vinol and dimethyl polysiloxane; oil such as live oil, cupu oil, carnauba wax, beef tallow, hardened oil, soybean oil, sesame oil, camellia oil, linseed oil, paraffin, liquid paraffin, cera flava, white vaseline, coconut oil and microcrystalline wax; fatty acid and its derivative such as stearic acid, aluminum stearate, calcium stearate, magnesium stearate, triethyl citrate, three ethyl ester medium chain fatty acid triglyceride, stearin and isopropyl myristate; alcohol and polyhydric alcohol such as ethyl alcohol, glycerinum, stearyl alcohol, cetyl alcohol, propylene glycol and polyethylene glycol; inorganic matter and metallic salt compound such as zinc oxide, calcium hydrophosphate, calcium carbonate, synthetic silicate, silicic anhydride, kaolin, dried aluminum hydroxide gel, synthetic hydrotalcites, titanium oxide, soapstone, bentonite, veegum, aluminium potassium sulfate, bismuth subgallate, bismuth subsalicylate, calcium lactate, sodium citrate, sodium chloride and sodium bicarbonate; surfactant such as sucrose fatty acid ester, polyhydrocarbon oxygen stearic acid ester, ethoxylated hydrogenated castor oil, polyoxyethylene polyoxypropylene glycol, doubleaccompanies sorbitan ester of oleic acid, three sorbitan ester of oleic acid, sorbitan monostearate ester, palmitic acid sorbitan ester, single lauric acid sorbitan ester, polysorbate, glyceryl monostearate, lauryl sodium sulfate and lauromacrogol; permeation enhancer such as dimethyl sulfoxide and its analogue, azone compound, pyrrolidone derivative, alcohol and fatty acid compound; pigment; perfume and so on.

Examples of interventional treatment device materials include injector, holder, artificial blood vessel, puncture syringe, catheter, saccule and so on.

The application of tanesthetic before the administration of the activating agent of the present invention depends on administration method, including injection anesthetics such as barbiturate, inhalation anesthetics such as nitrous oxide, superficial anesthesia such as lidocaine and so on.

The activating agent of the present invention can be applied to mammals with stem cells, such as human, monkey, dog, pig, cat, rabbit, rat and mouse, wherein human is preferred.

Most end stage cells in body system are ephemeral and must change continuously, wherein stem cells play a vital role (cell proliferation achieved by differentiated cells can reach the effect transitorily without stem cells); except for the normal cell exchange in body, the unexpected and sporadic tissue injury needs remedy of tissue regeneration initiated by stem cells, which can be induced by injury process. However, the condition that growing or adult animal loses most of the ability of building or regenerating tissues wit complete construction and function when issues are injured, which is the major pathogenic cause.

The target of regenerative medicine is to repair injured tissues and organs by regenerating tissues by utilizing proliferation, migration and differentiation of stem cells, especially the injured tissues and organs which cannot regenerate effectively by natural regenerative process.

A current research has found that stem cells are unevenly distributed in all tissues and organs in animal's body, including nerve tissues and heart tissues. In other words, it's believed that stem cells exist in all tissues and organs(Weissman IL. Translating stem and progenitor cell bilolgy to the clinic: Barriers and opportunities. Science. 287:1442-1446,2000 ; Garry DJ, et al. Ponce de Leon's fountain: stem cells and the regenerating heart. Am J Med Sci. 329(4): 190-201, 2005). In the field of regenerative medicine utilizing stem cell therapy, almost all of tissues in body have the examples of regeneration(Stem Cell Antholgy, Edited by Bruce M. Carlson, 2010, Elsevier Inc.). Skilled person in the field can summarize easily that almost all of the tissue injury diseases or functional disorder can be cured when the body tissues are regenerative(according to the injury degree of tissues) induced by specific environment of injured tissues if proliferation, migration and differentiation of stem cells can be initiated or activated.

The direct object of the present invention is to provide a method of activating proliferation, migration and differentiation of stem cells, which can be directly used for disease treatment requiring tissue regeneration and immunoregulation capability of stem cells. The mentioned diseases include the following two types: first, medicable disease simply treated by tissue regeneration of stem cells; second, medicable disease treated by tissue regeneration and immunoregulation capability of stem cells. In particular, the ins and outs(such as the effect of killing microorganism and removing slough played by chlorine dioxide oxidability) of the activating agent of the present invention are also used in the treatment of the above two diseases, and the range of the two diseases cover similar ranges in the serviceable range of the activating agent as a drug.

The following provides a detailed explanation of the fist disease(medicable disease simply treated by tissue regeneration of stem cells). In terms of the disease, skilled person in the field can summarize easily that the mentioned diseases can be cured by regenerative medicine utilizing stem cell therapy. Their curative mechanism is to repair the diseased tissues and organs by activating proliferation, migration and differentiation of normal stem cells (including resident stem cells, blood-derived stem cells and bone marrow-derived stem cells) in injured tissues.

There are particular limitations on the cause, type or degree of diseases (injured organs or injured tissues) to which regenerative medicine is applied. The followings are the specific examples of these diseases(the classifications of some specific diseases are partly repetitive).

The inventor of the present invention accidentally finds that the activating agent containing chlorine dioxide of the present invention has a positive effect on autoimmune diseases. Autoimmune disease covers most of anaphylactoid diseases and some clinically analogous agnogenic anaphylactoid diseases. The clinical diseases which are unable to be classified to some type of anaphylactoid disease or analogous agnogenic anaphylactoid disease are classified to other autoimmune diseases, which are included in the application scope of the present invention.

Other autoimmune diseases are also included in the application of the present invention: encephalomyelitis, Addison's disease, agammaglobulinemia, alopecia areata, amyotrophic lateral sclerosis, ankylosing spondylitis, antiphospholipid syndrome, anti-synthetase syndrome, aplastic anemia, autoimmune cardiomyopathy, autoimmune enteropathy, autoimmune hepatitis, autoimmune inner ear disease, autoimmune lymphoproliferative syndrome, autoimmune peripheral neuropathy, autoimmune pancreatitis autoimmune polyendocrinopathy syndrome, autoimmune progesterone dermatitis, autoimmune urticaria, BALO concentric sclerosis, Behcet's disease, thromboangiitis obliterans, bickerstaff encephalitis, Blau syndrome, bullous pemphigoid, castleman's disease, south American trypanosomiasis, chronic inflammatory demyelinating polyneuropathy, chronic recurrent multifocal osteomyelitis, chronic obstructive pulmonary disease, churg-strauss syndrome , cicatricial pemphigoid, Cogan's syndrome, complement component deficiency, cranial arteritis, CREST syndrome, cushing's syndrome, skin vasculitis leukocytes, malignant atrophic papulosis, Dercum's disease, dermatitis herpetiformis, dermatomyositis, diffuse cutaneous systemic sclerosis, dressler's syndrome, drug-induced lupus, eczema, endometriosis, enthesitis related arthritis, eosinophilic fasciitis, eosinophilic gastroenteritis, acquired epidermolysis bullosa, erythema nodosum, cryoglobulinemia, evan's syndrome, pulmonary fibrosis, gastritis, gastrointestinal pemphigoid, giant cell arteritis inflammation, allergic purpura, pregnancy pemphigoid, hidradenitis suppurativa, hughes-Stovin syndrome, low immune globulin, idiopathic inflammatory demyelinating disease, inclusion body myositis, chronic inflammatory demyelinating polyneuropathy, interstitial cystitis, juvenile rheumatoid arthritis, Lambert - Eaton syndrome, Kawasaki syndrome, leukocyte vasculitis, lichen planus, neurodermatitis, lichen sclerosus, linear IgA bullous dermatoses, amyotrophic lateral sclerosis, Majeed syndrome, mixed connective tissue disease, scleroderma, micro-polyarteritis nodosa, Guillain - Barre syndrome, pityriasis rosea, Ord's thyroiditis, palindromic rheumatism, paroxysmal nocturnal hemoglobinuria, Parry's syndrome, acute brachial plexus lesions, intermediate uveitis, periphlebitis purpura malignant, POEMS syndrome, Polyarteritis nodosa, polymyalgia rheumatica, polymyositis, primary biliary cirrhosis, primary sclerosing cholangitis, inflammatory lesions, gangrenous pyoderma, pure red cell aplasia, chronic focal encephalitis, Raynaud's phenomenon, relapsing polychondritis, reactive arthritis, retroperitoneal fibrosis, restless legs syndrome, autoimmune polyendocrinopathy syndrome type II , schnitzler syndrome, scleritis, Sjogren's syndrome, stiffman syndrome, sympathetic ophthalmia, aorto-arteritis, Susac's syndrome, Sydenham's chorea, Sweet's syndrome, Tolosa-Hunt syndrome, transverse spinal cord inflammation, undifferentiated connective tissue disease, vitiligo, Wegener's granulomatosis.

The followings are the description for the examples related to present invention.

In most cases, the potential technology can be applied. Although the followings provide a detailed explanation of the present invention by indicating examples thereof, the present invention is not limited to these examples.

**Example 1:**

The example is used to demonstrate the effect of activating agent of stem cells comprising a chlorine dioxide on androgenetic alopecia as a medicine.

Drug preparation: The first solution was a mixed solution made of sodium chlorite with the concentration of 7.47% and sodium chloride with the concentration of 1.59% with deionized water; the second solution was made of citric acid with the concentration of 16.7% with deionized water.

Dosage and administration: Same volume of solutions were taken out from different vessels with different volumes and put into the suitable glass or plastic cups prepared. Allow the solution to mix for 3-5 minutes and then filter the solution with $0.22\mu m$ filter membrane of double-deck. The alopecia area was smeared with the solution by swab, waiting for 1-2 hours before cleaning the head, once a day. The medicine was taken according to the course of treatment. The first course of treatment was 15-25 days, the second course of treatment began after 2-5 months of drug withdrawal. The administration days were 10-15 days from the second course of treatment, and the next course of treatment began after 2-5 months of drug withdrawal.

The subjects consisted of 42 male hair-loss patients and one female patient (22-68 years old.) They were willing to use the drug (hereinafter referred to as administration). See Table 1 for specific information.

Table 1

| No. effective patients | Effective rate | Description of the effect |
|---|---|---|
| 42 | 98% | 3-5 days after administration, the beard like new hair regrew, seemingly thick and strong, and can be seen by naked eyes. |
| 43 | 100% | 2-6 days after administration, the hair in the affected area fell off quickly with the thin hair fell off first. The serious the alopecia was, much hair it fell off. All hair in the alopecia area of the patients above level four would fall off after 6 days. |
| 40 | 93% | 15-25 days after administration, the new hair grew persistently. The hairline of the mild patients forward lead obviously. |
| 35 | 81% | It can be evaluated by naked eyes that the hair recovered to one level when the medicine was |
| | | stopped for 2-5 months after a course of treatment. Such as level 2 recovered to level 1 or level 4 recovered to level 3. Second course of treatment began after 1-2 months and it recovered to a new level, judging from naked eyes. |

From the fifth minutes, the patients felt stabbing pain that last for about 15-30 minutes; micro wound was produced in the area when being administered for three days. It was red spot, seemingly like the solidified bleeding wound. The new hair grew in that area was of higher density and became thicker.

To select four circular regions($5.1\ cm^2$) with diameter of 2.54cm in the following regions: the center of crown(in front of the whirl on the head, region 1), the middle of crown and forehead(region2), on the forehead(region 3), both sides of forehead(region 4), and calculate the amount of hair through digital photographs respectively on the 10th day (t1), the first month (t2), second month(t3) after administration of the first course of treatment, and the second month (t4) after administration of the second course of treatment, as well as the second month(t5) after administration of the third course of treatment. Then the increased ratios of hair density in different regions were worked out by comparing the amount of hair before administration. (See Table 2)

Table 2

| | T1 | T2 | T3 | T4 | T5 |
|---|---|---|---|---|---|
| Region 1 | 123% | 137% | 154% | 196% | 354% |
| Region 2 | 117% | 129% | 142% | 185% | 402% |
| Region 3 | 133% | 134% | 153% | 201% | 396% |
| Region 4 | 102% | 157% | 163% | 260% | 433% |

$P < 0.001$, n=43

According to the results shown in Table 1 and Table 2, activating agent of stem cells containing chlorine dioxide had far more significant effect on alopecia than those traditional medicines, such as Finasteride and minoxidil. The fundamental cause of the effect was that it solved the key to the question, that is, something wrong happened when the stem cells transforming into progenitor cells in hair-loss region. As a result, the stem cells failed to differentiate more follicle cells. (Luis A. Garza et al, Bald scalp in men with androgenetic alopecia retains hair follicle stem cells but lacks CD200-rich and CD34-positive hair follicle progenitor cells. J Clin Invest. 2011 February 1; 121(2): 613-622.). The activating effect of chlorine dioxide in the present invention was considered to be on promoting proliferation and migration of the follicle stem cells and/or follicle progenitor cells, so that new hair could grow obviously.

Furthermore, chlorine dioxide solution of acidity condition had offensive effect on skin, thus micro wound would be produced where the effect of growing hair was more obvious than other regions, perhaps the solution permeated completely and the growth promotion of the stem cells of chlorine dioxide could come into play; or it may indicate that new wound appeared or wound healing, and the stem cells differentiated according to original procedure, and more new hair would grow(patients were supposed to have.) Generally, follicle stem cell would not differentiate into epidermis cells for the natural renewal of skin. While if the skin wounded, the follicle stem cells would proliferate and migrate quickly to heal the wound. During the process, the follicle stem cells played a role in reepithelialization. (Ito M, Liu Y, Yang Z, Nguyen J, Liang F, Morris RJ, et al. Stem cells in the hair follicle bulge contributed to wound healing, but not to homeostasis of the epidermis. Nat Med. 11:1351-4.2005).

Besides, follicle stem cells can quicken wound healing, and the wound healing would delay without it. (Abigail K Langton et al. An Extended Epidermal Response Heals Cutaneous Wounds in the Absence of a Hair Follicle Stem Cell Contribution. Journal of Investigative Dermatology. 128, 1311-1318,2008). In the present example, the wound healing wasn't deferred, instead, it healed quickly. Combining with other aspects, it can be evaluated that the follicle stem cells completely participated in the process of wound healing and regrowing hair. For the research of the rats, new follicle stem cells group was produced during the process of artificial wound healing and rebirth of hair. For the research of the rats, new hair follicle stem cells communities were produced in the process of artificial wound healing and new hair also produced. Meanwhile, both follicle stem cell and epidermal stem cell participated in the formation of the cells(including hair follicle cells and sebaceous gland cells) in the wound healing process, among which the proliferation, migration and differentiation of the stem cells were included, and Wnt signals played important role. (Ito M, et al. Wnt-dependent de novo hair follicle regeneration in adult mouse skin after wounding. Nature. 2007, May 17;447(7142):316-20).

Combining these results with the theory analysis, it indicated that the effects of activating agent stem cells comprising chlorine dioxide was considered to be on promoting proliferation, differentiation and migration of various stem cells, so as to finish the process of tissue regeneration, such as wound healing and rebirth of hair. On the other hand, for most of patients, new hair could grow 2-3 days after administration, which can only be explained by stem cell differentiation. And the time of wound healing was almost the same with that of the general micro wound.

According to the example, the conclusion can be drawn by the skilled person in the field that the activating agent of stem cells in the present invention or the medicine made out of its main ingredients can be advantageously used in treating all kinds of skin diseases, and the mechanism of treatment is to promote tissue regeneration and repair diseased tissue and injured organs by activating stem cells.

## Example 2

In the present example, activating agent of stem cells comprising a chlorine dioxide was administered to patients with lower limb deep vein thrombosis (lower limb DVT), a kind of vascular disease, to evaluate the effects of activating agent of stem cells comprising a chlorine dioxide on activation of CD34-positive cells, CD34-positive/CD31-positive cells and VE-cadherin-positive cells in the peripheral blood, and the effects of activating agent of stem cells comprising a chlorine dioxide on regeneration of vessels damaged by thrombi.

Furthermore, those cells present in peripheral blood which are considered to be CD34-positive mononuclear cells are known to be vascular EPCs and mesenchymal stem cells (Zhao Y, et al. A human peripheral blood monocyte-derived subset acts as pleuripotent stem cells. Proc Natl Acad Sci USA 100: 2426-31, 2003). Thus, the CD34-positive mononuclear cells in the peripheral blood evaluated in the present example can be said to be vascular EPCs and mesenchymal stem cells.

In addition, endothelial progenitor cells are known to express CD34 (Michejda M. Which stem cells should be used for transplantation? Fetal Diagn Ther 19:2-8, 2004; Fadini G P, et al. Circulating endothelial progenitor cells are reduced in peripheral vascular complications of tape 2 diabetes mellitus. J American College of Cardiology 45:1449-1457, 2005; Kuwana M, et al. Human circulating CD14+ monocytes as a source of progenitors that exhibit mesenchymal cell differentiation. J Leukoc Biol 74:833-845, 2003) and CD31 (Asahara T, et al. Isolation of putative progenitor endothelial cells for angiogenesis. Science 275:964-967, 1997; Hristov M, Weber C. Endothelial progenitor cells: characterization, pathophysiology, and possible clinical relevance. J Cell Mol Med 8:498-508, 2004) as markers. Moreover, vascular EPCs, in which differentiation has progressed and are in a state of being able to adhere to blood vessels, are also known to further express VE cadherin (Hristov M, Weber C. Endothelial progenitor cells: characterization, pathophysiology, and possible clinical relevance. J Cell Mol Med 8:498-508, 2004). Thus, the CD34-positive/CD31-positive mononuclear cells and VE cadherin-positive mononuclear cells in peripheral blood evaluated in the present example can be said to be vascular EPCs.

Experimental Methods:

(1) Test Subjects

The subjects consisted of 14 male and 6 female lower limb DVT patients with the unilateral limb suffer from the illness, aged from 59 to 76 years old (average age: 67.2 years old). The purpose of the clinical test was explained to all subjects and their consent was obtained before conducting the clinical test.

(2) Medicament Preparation

The first solution was a mixed solution made of sodium chlorite with the concentration of 7.47% and sodium chloride with the concentration of 1.59% with deionized water; the second solution was made of citric acid with the concentration of 16.7% with deionized water. Same volume of solutions were taken out from different vessels with different volumes and mixed up. Allow the solution to mix for 3-5 minutes and dilute it 5 times with normal saline, then filter it with $0.22\mu$m filter membrane of double-deck. The solution was immediately put into injector. The solution was prepared once being used.

(3) Administration Method

Chlorine dioxide solution was administered to the patients through the contralateral femoral artery puncture (sick side) for 14 consecutive days, a dose of 10 ml /body/day. Before injection, use 2ml-5ml lidocaine of 2% to perform infiltration anesthesia at puncture point.

(4) Blood Sampling

Five ml of peripheral blood was collected from the median cubital vein of the forearm of each patient using low molecular weight heparin anticoagulant before administration (before initial administration) and on day 7 and 14 after administration, and the collected blood was immediately stored at 4° C. and measured within 6 hours after collection. Blood samples were collected from all 20 lower limb DVT patients before administration and on day 7 after administration, and from 15 of the 20 lower limb DVT patients on day 14 after administration.

(5) Separation of Peripheral Blood Mononuclear Cells

Two point five ml of lymphocyte separation liquid was added to 5 ml of the blood obtained in Experimental Method (4) above to prepare a suspension. After centrifuging the suspension at the condition of $460\times$g, the intermediate cell layer was recovered as the peripheral blood-derived mononuclear cells. The total number of the resulting mononuclear cells was about $5~6\times10^6$ cells per patient. The resulting mononuclear cells were suspended in phosphate-buffered saline (PBS) to prepare cell suspension with a concentration of $2\times10^6$ cells/ml.

(6) Isolation of CD34-Positive Mononuclear Cells

CD34-positive mononuclear cells were isolated by immunofluorescent stain using flow cytometry Twenty $\mu$l of phycoerythrin-labeled anti-CD34 antibody (Becton Dickinson, NJ, USA) was added to 100 $\mu$l of the mononuclear cell suspension obtained in Experimental Method (5) above and allowed to incubate for 30 minutes at 4° C. and under the dark condition. Next, CD34-positive mononuclear cells were isolated and counted by flow cytometry. The ratio (%) of CD34-positive mononuclear cells among the mononuclear cells was calculated according to the following formula.

$$\text{CD34-positive mononuclear cells (\%)} = (\text{No. of CD34-positive mononuclear cells/total No. of mononuclear cells}) \times 100$$

(7) Isolation of CD34-Positive /CD31-Positive Mononuclear Cells

CD34-positive mononuclear cells/CD31-positive mononuclear cells were isolated by double immunofluorescent using flow cytometry. Twenty $\mu$l of phycoerythrin-labeled anti-CD34 antibody and twenty $\mu$l of FITC-labeled anti-CD 31 were added to 100 μl of the mononuclear cell suspension obtained in Experimental Method (5) above and allowed to incubate for 30 minutes at 4° C. and under the dark condition. Next, CD34/CD31-positive mononuclear cells were isolated and counted by flow cytometry. The ratio (%) of CD34/CD 31-positive mononuclear cells among the mononuclear cells was calculated according to the following formula.

$$\text{CD34-positive mononuclear cells (\%)} = (\text{No. of CD34/CD 31-positive mononuclear cells/total No. of mononuclear cells}) \times 100$$

(8) Isolation of VE Cadherin-Positive Mononuclear Cells

VE Cadherin-positive mononuclear cells were isolated by immunofluorescent stain using flow cytometry. Twenty μl of FITC-labeled anti-VE cadherin antibody were added to 100 μl of the mononuclear cell suspensions obtained in Experimental Method (5) above and allowed to incubate for 30 minutes at 4° C. and under the dark condition. Next, VE cadherin-positive mononuclear cells were isolated and counted by flow cytometry. The ratio (%) of VE cadherin-positive mononuclear cells among the mononuclear cells was calculated according to the following formula.

$$\text{VE cadherin-positive mononuclear cells (\%)} = (\text{No. of VE cadherin-positive mononuclear cells/total No. of mononuclear cells}) \times 100$$

(9) Measurement of Girth of Above-Knee and Below-Knee

Lower limb edema is a typical and objective symptom of lower limb DVT. Therefore, the degree of lower limb edema is evaluated based on a lower limb girth in 20 cm of above-knee and in 15 cm of below-knee measured before and after administration of chlorine dioxide solution in the 20 patients.

Results and Discussion:

(1) Activation of CD34-Positive Mononuclear Cells

Table 3 is the changes of CD34-Positive Mononuclear Cells in Peripheral blood of lower limb DVT patients. (mean $\pm$ SD, %)

Table 3

|  | No. Patients | CD34-Positive Mononuclear Cells |
|---|---|---|
| Before administration | 20 | $0.38 \pm 0.42$ |
| Day 7 after administration | 20 | $0.91 \pm 0.51$** |
| Day 14 after administration | 15 | $1.4 \pm 0.68$** |
| **indicates $P < 0.01$ as compared with the before administration using the paired-sample non-parameter test. | | |

As a result of administration of chlorine dioxide solution, the percentage of CD34-positive mononuclear cells in the peripheral blood increased significantly as compared with the value of before administration (Table 3).
This result indicates that chlorine dioxide solution activated CD34-positive mononuclear cells (i.e., vascular EPCs and mesenchymal stem cells) of the peripheral blood in the lower limb DVT patients. The activating effect of activating agent of stem cells comprising a chlorine dioxide is considered to be on promoting proliferation and migration of the CD34-positive mononuclear cells.

(2) Activation of CD34-Positive/CD31-Positive Mononuclear Cells

Table 4 is the changes of CD34-Positive/CD31-Positive Mononuclear Cells in the Peripheral blood of lower limb DVT patients. (mean $\pm$ SD, %)

Table 4

| Time Period | No. Patients | CD34-Positive/CD31-P ositive Mononuclear Cells |
|---|---|---|
| Before administration | 20 | $0.28 \pm 0.30$ |
| Day 7 after administration | 20 | $0.85 \pm 0.51$** |
| Day 14 after administration | 15 | $1.1 \pm 0.65$** |
| **indicates P < 0.01 as compared with the before administration using the paired-sample non-parameter test. | | |

As a result of administration of chlorine dioxide solution, the percentage of CD34-positive/CD31-positive mononuclear cells in the peripheral blood increased significantly as compared with the value of before administration (Table 3).

This result indicates that chlorine dioxide solution activated CD34-positive/CD31-positive mononuclear cells (i.e., vascular EPCs and mesenchymal stem cells) of the peripheral blood in lower limb DVT patients. The activating effect of activating agent of stem cells comprising a chlorine dioxide is considered to be on promoting proliferation, migration and differentiation of CD34-positive/CD31-positive mononuclear cells.

(3) Activation of VE Cadherin-Positive Mononuclear Cells

Table 5 is the changes of VE cadherin-positive mononuclear cells in the peripheral blood of lower limb DVT patients (mean $\pm$ SD, %)

Table 5

| | No. Patients | VE Cadherin-Positive Mononuclear Cells |
|---|---|---|
| Before administration | 20 | $0.44 \pm 0.70$ |
| Day 7 after administration | 20 | $1.5 \pm 1.11$** |
| Day 14 after administration | 15 | $1.91 \pm 1.65$** |
| **Indicates P < 0.01 as compared with the before administration using the paired-sample non-parameter test. | | |

As a result of administration of chlorine dioxide solution, the percentage of VE cadherin-positive mononuclear cells in the peripheral blood increased significantly as compared with the value of before administration (Table 5).

This result indicates that chlorine dioxide solution activated VE cadherin-positive mononuclear cells (i.e., vascular EPCs) of the peripheral blood in lower limb DVT patients. The activating effect of activating agent of stem cells comprising a chlorine dioxide is considered to be on promoting proliferation, migration and differentiation of VE cadherin-positive mononuclear cells.

(4) Improvement of the Edema Symptoms of Lower Limb DVT

Table 6 is the changes of girth of above-knee and below-knee in lower limb DVT patients (mean $\pm$ SD, %)

Table 6

| | No. Patients | Girth of Above-Knee | Girth of Below-Knee |
|---|---|---|---|
| Before administration | 20 | $55.6 \pm 6.3$ | $38.2 \pm 4.3$ |
| Day 14 after administration | 20 | $50.7 \pm 5.5$* | $32.4 \pm 2.6$** |
| **indicates P < 0.01 as compared with the before administration using the paired-sample non-parameter test. | | | |

The degree of lower limb edema is evaluated based on a lower limb girth in 20cm of above-knee and in 15cm of below-knee. The girth of above-knee and below-knee decreased significantly by administration of chlorine dioxide solution (Table 6).

This result indicates that stem cells are activated by administration of chlorine dioxide solution which is considered to be on promoting proliferation, migration and differentiation of the stem cells, resulting in damaged vessels are regenerated and edema symptoms are improved in the lower limb DVT patients.

In addition, although the parameters of APTT, PT and TT, which serve as indicators of side effects were analyzed before administration, on day 7 and day 14 after administration of activating agent of stem cells comprising a chlorine dioxide, there were no significant differences between before and after administration of activating agent of stem cells comprising a chlorine dioxide. such as bleeding manifesting during administration of preparations acting on the blood coagulation and fibrinolysis systems (including activating agent of stem cells comprising a chlorine dioxide. These results indicate that there is no occurrence of side effects by administration of chlorine dioxide solution. However, almost all the patients felt stabbing pain that last for 30 minutes and then disappeared after injection without using anesthetic (day one after administration).

According to the example, the conclusion can be safely drawn by the skilled person of the field that the activating agent of the stem cells comprising chlorine dioxide or the medicines that made of the main compositions in the invention could be used to treat all kinds of cardiovascular and cerebrovascular diseases. The mechanism of the treatment is to promote regeneration of tissue and repair the diseased tissue as well as injured organs by activating the stem cell.

**Example 3**

The example is to demonstrate the effects of activating agent of stem cells comprising a chlorine dioxide on activation of Neural Stem Cells and Recovery of Neural Function in a Cerebral Ischemia/Reperfusion Injury Model.

(1)Experimental Subject:

Male Sprague-Dawley rats with the age of 12 weeks and weight of 250~280g were used in the experiment after acclimating for a week.

(2)Establishment of Cerebral Ischemia/Reperfusion Injury Model

A middle cerebral artery occlusion model was first established in compliance with the method of Longa E Z et al. (Longa E Z, et al. Reversible middle cerebral artery occlusion without craniectomy in rats. Stroke 20:84-91, 1989). More specifically, 0.36 g/kg of 10% chloral hydrate was administered intraperitoneally to rats which had been fasted for 12 hours (although given free access to water) to anesthetize the rats. Next, a midline incision was made in the neck to expose the right common carotid artery. Then, the internal carotid artery and external carotid artery were separated, the posterior carotid artery, which is a branch of the external carotid artery, and the thyroidea arteria were coagulated and severed, the external carotid artery was ligated at the branch of the lingual artery and maxillary artery, a small puncture was formed in the external carotid artery, 4-0 Nylon suture was passed through the common carotid artery from the puncture, and gradually inserted into the internal carotid artery until there was resistance. The length of the Nylon suture was about 18~20 mm from the branch of the common carotid artery. As a result of occluding the middle cerebral artery with Nylon suture, an ischemic state was applied for 2 hours to create a middle cerebral artery occlusion model.

A cerebral ischemia/reperfusion injury model was established by removing the Nylon suture from the middle cerebral artery of the occlusion model and following reperfusion of blood.

(3) Drug Preparation Methods

The first solution was a mixed solution made of sodium chlorite with the concentration of 7.47% and sodium chloride with the concentration of 1.59% with deionized water; the second solution was made of citric acid with the concentration of 16.7% with deionized water. Same volume of solutions were taken out from different vessels with different volumes and mixed up. Allow the solution to mix for 3-5 minutes and dilute it five times with normal saline, and then filter it with $0.22\mu m$ filter membrane of double-deck. The solution was immediately put into injector. The solution was prepared once being used.

(4) Administration of activating agent of stem cells comprising a chlorine dioxide to Model Rats

The rats were divided into three groups and respectively treated in the manner described below.

Treatment group (120 rats): A cerebral ischemia/reperfusion injury model was established for the rats already. To administer chlorine dioxide solution everytime that prepared from procedure (3) through carotid artery after the second day the model has established, once a day for total ten days.

Sham-operation group (120 rats): The above-mentioned procedure (2) was performed in the rats just for separation of the internal carotid artery and external carotid artery, subsequent occlusion of the middle cerebral artery and reperfusion procedures were not performed. The rats in this group were administered same volume of physiological saline alternative via carotid artery instead of chlorine dioxide solution on the same times of administration as indicated in experimental group.

Model group (120 rats): These animals were used to establish the cerebral ischemia/reperfusion injury model. The model rats in this group were administered same volume of physiological saline alternative via carotid artery instead of chlorine dioxide solution on the same times of administration as indicated in treatment group.

(5) Evaluation of Neural Function Score

Neural function scores were evaluated once a day after the ischemia/reperfusion injury model established in the 15 rats per group. The scores of neural function disorders were evaluated using the following criteria in compliance with the method of Longa E Z (Longa E Z, et al. Reversible middle cerebral artery occlusion without craniectomy in rats. Stroke 20:84-91, 1989). The results were shown as median.

0: Normal
1: Contralateral limb bending on infarction focus
2: Contralateral limb on infarction focus becomes asthenic state when tail is pulled backward
3: Turning toward opposite side of infarction focus when tail is pulled
4: Spontaneous turning towards opposite side of infarction focus
5: Loss of spontaneous movement

(6) Analysis of Nestin-Positive Cells

Ten rats of each group were autopsied on day 7, 14 and 21 after the model established, respectively (total animals are 30 rats from each group). After preparation of tissue specimens, the neural stem cells were counted under a microscope.

More specifically, rat frozen brain sections were prepared on day 7, 14 and 21 after the model established, and the presence of nestin-positive cells in the subventricular zone (SVZ) was measured by immunohistochemical stain using the marker of nestin expressed on the neural stem cells. The presence of nestin-positive cells was analyzed using mouse anti-rat nestin monoclonal antibody.

(7) Analysis of BrdU-Positive Cells and BrdU-Positive/Nestin-Positive Cells

On day 5, 6, 12, 13, 19 and 20 after the model established, 50 mg/kg of 5-bromodeoxyuridine was injected intraperitoneally into 30 rats in each group. Because BrdU is a thymidine analog, it can incorporate into the DNA of cells during the course of cell proliferation (DNA synthesis). Thus, cell proliferation can be analyzed by detecting the amount of intracellular BrdU. The rats were autopsied on day 7, 14 and 21 after the model established for preparation of frozen brain sections. The presence of BrdU-positive cells by immunochemical stain, and the presence of BrdU-positive/nestin-positive cells by double-immunofluorescent stain in the SVZ region were analyzed.

The presence of BrdU-positive cells was analyzed using mouse anti-rat BrdU monoclonal antibody.

The presence of BrdU-positive/nestin-positive cells was analyzed by double-immunofluorescent stain using a combination of primary antibody of goat anti-rat BrdU polyclonal antibody and secondary antibody of FITC-labeled rabbit anti-goat antibody, and a combination of primary antibody of mouse anti-rat nestin monoclonal antibody and secondary antibody of Rho-labeled rabbit anti-mouse antibody.

(8) Analysis of NeuN-Positive Cells, GFAP-Positive Cells, and BrdU-Positive/GFAP Positive Cells

On day 5, 6, 12, 13, 19 and 20 after the model established, 50 mg/kg BrdU was injected intraperitoneally into 30 rats in each group. The rats were autopsied on day 7, 14 and 21 after the model established for preparation of frozen brain sections. The presences of NeuN-positive cells by immunochemical stain, glial fibrillory acidic protein (GFAP)-positive cells by immunochemical stain, and BrdU-Positive/GFAP Positive Cells by double-immunofluorescent stain in the SVZ region were analyzed. NeuN is a marker expressed on the immature neurons which are differentiated from neural stem cells. GFAP is a marker expressed on astrocytes which are also differentiated from neural stem cells.

The presence of NeuN-positive cells was analyzed using goat anti-NeuN monoclonal antibody. The presence of GFAP-positive cells was analyzed using goat anti-GFAP polyclonal antibody. The presence of BrdU-positive/GFAP-positive cells was analyzed by double-immunofluorescent stain using a combination of primary antibody of mouse anti-rat BrdU monoclonal antibody and secondary antibody of Rho-labeled rabbit anti-mouse antibody, and a combination of primary antibody of goat anti-GFAP polyclonal antibody and secondary antibody of FITC-labeled rabbit anti-goat antibody.

(9) Analysis of BrdU-Positive (Migrating) Cells

Thirty Rats in each group were intraperitoneally injected twice with 50 mg/kg of BrdU within 24 hours after model established. The rats were autopsied on day 7, 14 and 21 after model established for preparation of frozen brain sections. The presence of BrdU-positive cells in the SVZ region was analyzed by immunochemical stain using mouse anti-rat BrdU monoclonal antibody.

Results and Discussion:

(1) Evaluation of Neural Function

The scores were detected on every day after the ischemia/reperfusion injury model established until day 21 of terminal of the experiment. Table 7 is the changes of neural function scores on time course (n = 15, median).

Table 7

| Day | 1 | 2 | 3 | 4 | 5 | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|---|---|---|---|---|
| Sham-operation | 0 | 0 | | 0 | 0 | 0 | 0 | 0 | 0 | 0 | 0 | 0 | 0 | 0 | 0 | 0 | 0 | 0 | 0 | 0 | 0 |
| Group model | 3 | 3 | 3 | 2 | 2 | 2 | 2 | 2 | 1 | 1 | 1 | 1 | 1 | 1 | 1 | 1 | 1 | 1 | 1 | 1 | 1 ** |
| Experimental Group | 3 | 3 | 2 | 2 | 1 | 1 | 1 | 0 | 0 | 0 | 0 | 0 | 0 | 0 | 0 | 0 | 0 | 0 | 0 | 0 | 0 ** |

**indicates $P < 0.05$ as compared with the day 1 of each group using the Kruskal-Wallis test.

In the sham-operation group, the neural function disorders were not observed. In the other two groups, significant recovery of neural function was observed on day 21 compared with that on day 1 after the operation ($P<0.05$).

In the treatment group, recovery of neural function was observed to occur earlier than that in the model group. Significant recovery of neural function was observed in the treatment group particularly on day 7, 8, 9 and 16 after operation compared with the model group.

This result indicates that chlorine dioxide solution activated neural stem cells, resulting in contributing to the improvement of neural function disorders. The activating effect of activating agent of stem cells comprising a chlorine dioxide is considered to be on promoting proliferation, migration and differentiation of the neural stem cells.

(2) Demonstration of the Presence of Neural Stem Cells

Table 8 is the numbers of nestin-positive cells in the SVZ region (mean $\pm$ SD, No. cells/mm2)

Table 8

| | Day 7 | Day 14 | Day 21 |
|---|---|---|---|
| Sham-operatio n | 45.6±5.69 | 13.2±1.64 | 1.99±1.09 |

(continued)

|  | Day 7 | Day 14 | Day 21 |
|---|---|---|---|
| Model group | 210.5±22.3* | 74.5±13.4* | 46.2±6.31* |
| Treatment group | 305.2±30.6** | 105.3±15.4** | 63.2±7.55** |
| BRt | 157.4% | 150.2% | 138.5% |
| **Indicates P < 0.05 as compared with the sham-operation group, *indicates P< 0.01 as compared with the model group on the same day using the Wilcoxon's test. | | | |

According to the data shown in Table 8, the relative value (%) of the number of nestin-positive cells in the treatment group was calculated based on a value of 100% for the model group using the following formula:

$$BRt(\%)=(Bt-St)/(Mt-St)\times100$$

BRt(%): Percentage of nestin-positive cells in treatment group at time t
Bt: Mean number of nestin-positive cells in treatment group at time t
St: Mean number of nestin-positive cells in sham-operation group at time t
Mt: Mean number of nestin-positive cells in model group at time t
t: Day 7, day 14 or day 21

In comparison with the sham-operation group, the number of nestin-positive cells increased in the model group (nestin is a marker for neural stem cells) (Table 8). This is considered to be attributable to a compensatory reaction induced by stimulation of the cerebral ischemia/reperfusion injury in the animals.
On the other hand, the numbers of nestin-positive cells increased in the treatment group as compared with the model group on each of day 7, 14 and 21 after model established (Table 8).
These results indicate that chlorine dioxide solution activated the nestin-positive cells (i.e., neural stem cells). The activating effect of chlorine dioxide solution is considered to be on promoting proliferation and migration of the neural stem cells.
In addition, although chlorine dioxide solution was not administrated from day 11 after model established, the numbers of nestin-positive cells significantly increased in the treatment group as compared with the model group on day 21 after model established. This result indicates that activating agent of stem cells comprising a chlorine dioxide has a sustainable activating effect on neural stem cells.

(3) Demonstration of Presence of Proliferating Neural Stem Cells and Their Self-Replication Ability

a. Demonstration of the Presence of Proliferating Neural Stem Cells

The presence of proliferating nestin-positive/BrdU-positive cells (i.e., neural stem cells) were confirmed by double-immunofluorescent stain in the SVZ region in the treatment group. This indicates the presence of proliferating neural stem cells in the treatment group.

b. Demonstration of the Self-Replication Ability of Neural Stem Cells

Table 9 shows the numbers of BrdU-positive cells in the SVZ region (mean ± SD, No. cells/mm2)

Table 9

|  | Day 7 | Day 14 | Day 21 |
|---|---|---|---|
| Sham-operation | 52.8±3.85 | 33.9±2.95 | 23.9±1.95 |
| Model group | 136.56±9.93* | 328.6±12.5* | 279.9±12.8* |
| Treatment group | 184.61±12.3** | 496.2±30.6** | 385.3±20.9** |

(continued)

|  | Day 7 | Day 14 | Day 21 |
|---|---|---|---|
| BRt | 157.4% | 156.9% | 141.2% |
| *Indicates P < 0.01 as compared with the sham-operation group, **indicates P < 0.01 as compared with the model group on the same day using the Wilcoxon's test. | | | |

According to the data shown in Table 9, the relative value (%) of the number of BrdU-positive cells in the treatment group was calculated based on a value of 100% for the model group using the following formula:

$$BRt(\%)=(Bt-St)/(Mt-St)\times100$$

BRt(%): Percentage of BrdU-positive cells in treatment group at time t
Bt: Mean number of BrdU-positive cells in treatment group at time t
St: Mean number of BrdU-positive cells in sham-operation group at time t
Mt: Mean number of BrdU-positive cells in model group at time t
t: Day 7, day 14 or day 21

In comparison with the sham-operation group, the number of BrdU-positive cells increased in the model group (BrdU is a marker for proliferating cells) (Table 9). This is considered to be attributable to a compensatory reaction induced by stimulation of the cerebral ischemia/reperfusion injury in the animals.

On the other hand, the numbers of BrdU-positive cells increased in the treatment group as compared with the model group on each of day 7, 14 and 21 after model established (Table 9.)

BrdU-positive cells present in brain tissue consist only of neural stem cells and neural progenitor cells which are capable of incorporating BrdU based on their self-replication ability. Thus, these results indicate that activating agent of stem cells comprising a chlorine dioxide activated neural stem cells and/or neural progenitor cells. The activating effect of chlorine dioxide solution is considered to be on promoting proliferation and migration of the neural stem cells and/or neural progenitor cells.

In addition, although chlorine dioxide solution was not administrated from day 11 after model established, the numbers of BrdU-positive cells significantly increased in the treatment group as compared with the model group on day 21 after model established. This indicates that activating agent of stem cells comprising a chlorine dioxide has a sustainable activation effect on neural stem cells and/or neural progenitor cells.

(4) Demonstration of the Presence of Immature Neurons, Astrocytes Differentiated from Neural Stem Cells and the Astrocytes Increased

a. Demonstration of the Presence of Immature Neurons

The presence of NeuN-positive cells was confirmed in the treatment group (data not shown). NeuN is a marker expressed on the immature neurons which are differentiated from neural stem cells. Thus, this result indicates that immature neurons differentiated from neural stem cells are present in the treatment group.

b. Demonstration of Presence of Astrocytes Differentiated from Neural Stem Cells

The presence of BrdU-positive/GFAP-positive cells was confirmed using double-immunofluorescent stain in the SVZ region in the treatment group (data not shown). GFAP is a marker expressed on astrocytes which are differentiated from neural stem cells. Thus, this result indicates that astrocytes differentiated from neural stem cells are present in the treatment group. However, astrocytes are differentiated cells in the neural tissues, why BrdU was incorporated into them? It is considered that the detected BrdU is incorporated into the cells when in the state of stem cells and/or progenitor cells prior to differentiating into astrocytes, and existed continuously.

c. Demonstration of the Presence of the Astrocytes Increased

Table 10 shows the numbers of GFAP-positive cells in the SVZ ((mean $\pm$ SD, No. of cells/mm2)

Table 10

| | Day 7 | Day 14 | Day 21 |
|---|---|---|---|
| Sham-operation group | 2.5±0.15 | 1.39±0.02 | 0 |
| Model group | 66.3±21.3* | 58.6±16.5* | 27.9±12.4* |
| Treatment group | 97.2±29.3** | 78.2±19.6** | 45.3±18.3** |
| BRt | 148.4% | 134.3% | 162.4% |
| **Indicates P < 0.05 as compared with the sham-operation group, *indicates P < 0.01 as compared with the model group on the same day using the Wilcoxon's test. | | | |

According to the data shown in Table 10, the relative value (%) of the number of GFAP-positive cells in the treatment group was calculated based on a value of 100% for the model group using the following formula.

$$BRt(\%) = (Bt - St)/(Mt - St) \times 100$$

BRt(%): Percentage of GFAP-positive cells in treatment group at time t
Bt: Mean number of GFAP-positive cells in treatment group at time t
St: Mean number of GFAP-positive cells in sham-operation group at time t
Mt: Mean number of GFAP-positive cells in model group at time t
t: Day 7, day 14 or day 21

In comparison with the sham-operation group, the number of GFAP-positive cells increased in the model group (GFAP is a marker for astrocytes differentiated from neural stem cells) (Table 10). This is considered to be attributable to a compensatory reaction induced by the stimulation of cerebral ischemia/reperfusion injury in the animals.

On the other hand, the numbers of GFAP-positive cells increased in the treatment group as compared with the model group on each of day 7, 14 and 21 after model established (Table 10.)

These results indicate that chlorine dioxide solution activated the GFAP-positive cells (i.e., neural stem cells and/or neural progenitor cells). Therefore, the activating effects of chlorine dioxide solution is considered to be on promoting proliferation, differentiation and migration of neural stem cells and/or neural progenitor cells, and particularly on promoting differentiation to astrocytes from above cells.

In addition, although activating agent of stem cells comprising a chlorine dioxide was not administrated from day 9 after model established, the numbers of GFAP-positive cells increased in the treatment group as compared with the model group on day 21 after model established. This result indicates that chlorine dioxide solution has a sustainable activating effect on neural stem cells and/or neural progenitor cells to differentiate into astrocytes.

(5) Demonstration of the Presence of the Migrating Cells

Table 11 shows the numbers of BrdU-positive cells in the SVZ region (mean ± SD, No. cells/mm2)

Table 11

| | Day 7 | Day 14 | Day 21 |
|---|---|---|---|
| Sham-group | 2.85±1.05 | 3.39±1.35 | 3.11±1.05 |
| Model group | 136.56±15.63* | 78.6±8.5* | 34.9±5.48* |
| Treatment group | 164.61±16.3** | 112.2±10.6** | 75.3±8.39** |
| BRt | 121.0% | 144.7% | 227.1% |
| *Indicates P < 0.01 as compared with the sham-operation group, **indicates P < 0.01 as compared with the model group on the same day using the Wilcoxon's test. | | | |

According to the data in Table 11, the relative value (%) of the number of BrdU-positive cells in the treatment group was calculated based on a value of 100% for the model group using the following formula.

$$BRt(\%)=(Bt-St)/(Mt-St)\times 100$$

BRt(%): Percentage of BrdU-positive cells in treatment group at time t
Bt: Mean number of BrdU-positive cells in treatment group at time t
St: Mean number of BrdU-positive cells in sham-operation group at time t
Mt: Mean number of BrdU-positive cells in model group at time t
t: Day 7, day 14 or day 21

In this experiment, BrdU was intraperitoneally administered only twice within 24 hours after model established, and since BrdU was not administered after that time, the BrdU-positive cells analyzed on day 7, 14 and 21 after model established are the BrdU-positive cells presented at the BrdU-administered time. Thus, increases in the number of BrdU-positive cells analyzed in the SVZ region on day 7, 14 and 21 after model established reflect the number of BrdU-positive cells which have migrated into the SVZ region. Moreover, BrdU-positive cells in the SVZ region consist only of neural stem cells and neural progenitor cells capable of incorporating BrdU based on the self-replication ability. On the basis of the above, Table 11 and FIG. 4 indicate the migration status of neural stem cells and neural progenitor cells into the SVZ region.

In comparison with the sham-operation group, the number of BrdU-positive cells in the model group increased (Table 11). This is considered to be attributable to a compensatory reaction induced by stimulation of the cerebral ischemia/reperfusion injury in the animals.

On the other hand, the numbers of BrdU-positive cells increased in the treatment group as compared with the model group on day 7, 14 and 21 after model established (Table 11). This indicates that the number of neural stem cells and/or neural progenitor cells which migrated to the SVZ region in the treatment group was larger than in the model group.

These results indicate that activating agent of stem cells comprising a chlorine dioxide activated neural stem cells. The activating effect of stem cell is considered to be on promoting migration of the neural stem cells and/or neural progenitor cells.

In addition, although chlorine dioxide solution was not administrated from day 11 after model established, the numbers of BrdU-positive cells increased in the treatment group as compared with the model group even on day 21 after model established. This result indicates that activating agent of stem cells comprising a chlorine dioxide has a sustainable activating effect on migration of neural stem cells and/or or neural progenitor cells.

Furthermore, the distribution of BrdU-positive cells in SVZ region was compared between the model group and treatment group by immunochemical stain. In the model group, BrdU-positive cells were only distributed on the surface of the SVZ region, but the BrdU-positive cells were widely distributed from the surface to interior of the SVZ region in the treatment group. This indicates that neural stem cells and/or neural progenitor cells migrated from the surface to the interior of the SVZ region as a result of administration of activating agent of stem cells comprising a chlorine dioxide, namely that activating agent of stem cells comprising a chlorine dioxide promoted migration of neural stem cells and/or neural progenitor cells in situ of the brain tissue.

These results in Example 3 clearly indicate that activating agent of stem cells comprising a chlorine dioxide has sustainable activating effects on neural stem cells. The activating effects of activating agent of stem cells comprising a chlorine dioxide are considered to be on promoting proliferation, migration and differentiation of the neural stem cells and/or neural progenitor cells.

According to the example, the conclusion can be drawn by the skilled person that activating agent of stem cells comprising a chlorine dioxide or the medicine made out of the activating agent are capable of treating nervous system disease or motor system disease. Besides, the mechanism of treatment is to promote tissue regeneration and repair diseased tissues and organs by activating stem cells.

## Example 4

The example is used to demonstrate the effects of activating agent of stem cells comprising a chlorine dioxide on alopecia areata.

The first solution was a mixed solution made of sodium chlorite with the concentration of 7.47% and sodium chloride with the concentration of 1.59% with deionized water; the second solution was made of citric acid with the concentration of 16.7% with deionized water.

Same volume of solutions were taken out from different vessels with different volumes and put into the suitable glass or plastic cups prepared. Allow the solution to mix for 3-5 minutes and then filter the solution with $0.22\mu m$ filter membrane of double-deck, dimethyl sulfoxide was added in a ratio of 1:1, and then smear solution over the alopecia areata with the swab, waiting for 1-2 hours before cleaning the head, the drug was administered once a day according to the method. The first period lasted for 15-25 days, then the drug was stopped for 2-5 months; The second period lasted for 15-25 days in the light of the situation.

The subjects consisted of 11 male alopecia areata patients and a female patient (the patients are between 22-40 years old.) They administered the activating agent of stem cell as volunteers, see Table 12.

Table 12

| Course of disease | No. patients | First period of administration | Second period of administration | Half a year after administration |
|---|---|---|---|---|
| Within 12 months | 5 | New hair equably grew 3-5 days after administration, and administration time was 15-25 days, the growth rate of the new hair was about 80%-90% after stopping the drug for a month. | About 50% of hair fell off, and new hair grew 3-5 days after administration. The administration time was 15-25 days, the growth rate was about 90%-95% after stopping the drug for a month. | Fully recovery |
| More than 12 months | 7 | New hair grew along the alopecia areata area 3-5 days after administration, the growth rate of the new hair was about 40%-50% after stopping the drug for a month. | Almost 50%-60% of hair fell off, new hair equably grow 3-5days. The growth rate was about 80%-90% after stopping the drug for a month. | Basic recovery Recovery rate was 90%-100%. |

Alopecia areata is the common disease of dermatological department, especially in young adults with different numbers of round or elliptic spots on the scalp. Alopecia areata is an autoimmune disease due to the body's failure to recognize "self" and destroys its own tissue as if it were an invader. The hair follicle stem cells are not destroyed, the hair can regrow. For almost 80% of the patient, new hair can grow 12 months after onset of the disease. But generally, it will attack repeatedly. Limited success has been shown from treating AA with corticosteroid injections, aiming at stimulating the hair to grow, but easy to recur.

The present example indicates that activating agent of stem cells comprising a chlorine dioxide is capable of repairing the immunoreaction of AA, so as to regrow hair follicle that can cure AA.

According to the example, the conclusion can be drawn by the skilled person in the field that the activating agent of stem cells comprising a chlorine dioxide or the medicine made of its main ingredients can be advantageously used in treating all kinds of autoimmune skin diseases and the mechanism of treatment is to promote tissue regeneration, regulate immune mechanism and repair injured tissues and organs by activating stem cells.

**Example 5**

The example is used to demonstrate the effects of activating agent of stem cells comprising a chlorine dioxide on osteoarthritis as a medicine.

The purpose of the experiment is to evaluate the effects of activating agent of stem cells comprising a chlorine dioxide on hip arthritis for intra-articular by evaluating the thickness of cartilage, pain intensity and life quality of the patients before and after the treatment.

The first solution was a mixed solution that made of sodium chlorite with the concentration of 8% and sodium chloride with the concentration of 2% with deionized water; the second solution was made of citric acid with the concentration of 15% with deionized water.

Same volume of solutions were taken out from different vessels with different volumes and put into the suitable glass or plastic cups prepared. Allow the solution to mix for 3-5 minutes and then filter the solution with $0.22\mu m$ filter membrane of double-deck, dimethyl sulfoxide was added in a ratio of 1:1. To smear the solution over the skin of hip arthritis with swab immediately, once a day for consecutive 20 days. Then stop the administration for three months, and then began continuous treatment for 20 days. Clean and disinfect the regions before treatment, conduct local anesthetic 30 minutes before the operation. Corresponding amount of solution was administered according to different regions, varying from $1-10ml/100cm^2$.

The subjects who joined in the treatment group as volunteers consisted of 11 patients with hip arthritis (5 male, aged from 40 to 70 years old and 6 female, aged from 40 to 65 years old.). Case histories were recorded at the third and the sixth month by collecting personal and data of human body (height, body weight, BMI, blood pressure and heart rate.) Then evaluate the following parameters:

Pain intensity: VAS was used, ranging from 0(normal) to 100(maximum intensity), it was conducted by the doctor and patient:

Seriousness of the illness: Womac, namely, self-assessment of hip arthritis that consists of 24 items was used to detect the development and effect of the treatment.

Thickness of cartilage of hip joint: measured by ultrasonic scanning at the core, the center and the lateral.

Results of the example:

The activating agent of stem cells comprising chlorine dioxide was tolerant enough without local or systemic anaphylactic reaction. During the administration, the patients felt continuous painful and itchy, but it disappeared completely about an hour.

For the data about pain intensity and seriousness of the illness, see Table 13.

(1)Evaluations of the pain intensity The VAS evaluated by the patients was 58.32 ($\pm$8.25) at the beginning of the example; the mean reached 18.24 ($\pm$7.25 )three months later and the percentage was down to 68% ($p < 0.05$), the mean will be 6.32 ($\pm$6.53) six months later and the percentage was down to 89.16% ($p < 0.05$). The VAS evaluated by the doctor was 55.23 ($\pm$9.66) at the beginning of the example; the mean was 20.03 ($\pm$7.32) and the percentage was down to 63.7% ($p < 0.05$); the mean was reduced to 7.11 ($\pm$6.93) six months later, and the percentage was down to 87.12% ($p < 0.05$).

(2)Evaluation of the seriousness of the illness The mean of WOMAC was 55.03 ($\pm$26.63) at the beginning of the experiment; the mean was reduced to 20.11 ($\pm$22.53), and the percentage was down to 59.06% ($p < 0.01$) three months later; the mean was reduced to 6.54 ($\pm$5.86) and the percentage was down to 88.12 ($p < 0.01$) six months later.

Table 13 is the clinical assessment of the patients evaluated at the beginning of the experiment and the third and sixth month after penetration treatment. Table 14 is the percent reduction in clinical assessment at the third and sixth month after the penetration treatment.

Table 13

| Assessment items | The very beginning of the administration | The third month | The sixth month |
|---|---|---|---|
| Patients VAS mean ($\pm$SD) | 58.32 ($\pm$8.25) | 18.24 ($\pm$7.25) | 6.32 ($\pm$6.53) |
| Doctor VAS mean ($\pm$SD) | 55.23 ($\pm$9.66) | 20.03 ($\pm$7.32) | 7.11 ($\pm$6.93) |
| WOMAC mean ($\pm$SD) | 55.03 ($\pm$26.63) | 20.11 ($\pm$22.53) | 6.54 ($\pm$5.86) |

Table 14

| Assessment items | The third month comparing with the before administration | The sixth month comparing with the before administration |
|---|---|---|
| Patients VAS mean ($\pm$SD) (using Wilcoxon's test) | 68.7% ($p < 0.05$) | 89.16% ($p < 0.05$) |
| Doctor VAS mean ($\pm$SD) (using Wilcoxon's test) | 63.7% ($p < 0.05$) | 87.12% ($p < 0.05$) |
| WOMAC mean (using Wilcoxon's test) | 59.06% ($p < 0.01$) | 88.12 ($p < 0.01$) |

(3)Thickness of joint cartilage

The ultrasound evaluation of cartilage(see Table 15)showed that the mean of middle cartilage before the administration was 0.51 ($\pm$0.07) mm; three months later, it increased to 0.59 ($\pm$0.08) mm, the percentage increased by 15.6% ($p < 0.05$); six months later, it increased to 0.66 ($\pm$0.16) mm, percentage increased to 11.9% ($p < 0.05$).

The mean of lateral cartilage before administration was 0.61 ($\pm$0.09) mm; three months later, it increased to 0.67 ($\pm$0.10) mm already, the percentage increased to 9.8% ($p < 0.05$); six months later, the thickness increased to

0.73 ($\pm$0.11) mm, and the percentage increased to 8.9% (p < 0.05).

The mean of central cartilage before administration was 0.73 ($\pm$0.09) mm; three months later, it increased to 0.79 ($\pm$0.11) mm, and the percentage increased to 8.2% (p < 0.05); six months later, it increased to 0.89 ($\pm$0.09) mm, and the percentage increased to 12.7% (p < 0.05).

Table 15 is about the evaluation of the thickness of key cartilage at the beginning and at the third and sixth month after penetration treatment

Table 15

| Evaluation items | Before administration | The third month comparing with the before administration | The sixth month comparing with the before administration |
|---|---|---|---|
| Medical cartilage mean($\pm$SD)/mm | 0.51 ($\pm$0.07) | 0.59 ($\pm$0.08) * 15.6% | 0.66 ($\pm$0.16) * 11.9% |
| Lateral cartilage mean($\pm$SD)/mm | 0.61 ($\pm$0.09) | 0.67 ($\pm$0.10) * 9.8% | 0.73 ($\pm$0.11) * 8.9% |
| Central cartilage ($\pm$SD)/m m | 0.73 ($\pm$0.09) | 3.19 ($\pm$0.7) * 8.2% | 0.89 ($\pm$0.09) * 12.7% |
| * indicates P < 0.05 as compared with the before administration using the Wilcoxon's test | | | |

The results analysis indicates that the patients had a clinically significant reduction in symptoms after administering the activating agent of stem cell comprising a chlorine dioxide. And the improvement that merely appears at the third month could maintain to the sixth month when the symptom of bone joint improved further. The pain intensity of VAS at the third and sixth month had a significant reduction compared with that before administration, which indicate that the medicine not only promote the viscoelasticity of synovia, but also can relieve pain obviously. Therefore, it has a substantial improvement for the life quality of the patients. That is to say, the activating agent of stem cell comprising a chlorine dioxide is capable of regenerating inflammation tissue remarkably.

According to the example, the conclusion can be drawn by the skilled person in this field that the activating agent of stem cells in the present invention or the medicine made of its main ingredients can be advantageously used in treating all kinds of inflammatory diseases and motor system diseases. Besides, the mechanism of treatment is to promote tissue regeneration and repair injured tissues and organs by activating stem cells.

**Example 6**

The example is used to demonstrate the effects of activating agent of stem cells comprising a chlorine dioxide on Ankylosing spondylitis.

Ankylosing spondylitis is a chronic, progressive and disabling autoimmune disease of the axial skeleton. The main cause for disablement is variable involvement of peripheral joints. The purpose of the example is to evaluate the effects of activating agent of stem cells comprising a chlorine dioxide on the autoimmune disease--AS.

The first solution was a mixed solution made of sodium chlorite with the concentration of 7.47% and sodium chloride with the concentration of 1.59% with deionized water; the second solution was made of citric acid with the concentration of 16.7% with deionized water. Same volume of solutions were taken out from different vessels with different volume and put into the suitable glass or plastic cups prepared. Allow the solution to mix for 3-5 minutes and then filter the solution with 0.22$\mu$m filter membrane of double-deck, dimethyl sulfoxide was added in the ratio of 1:1. The solution was smeared over the region immediately and repeatedly, once a day for consecutive three weeks. The administration was stopped for a week, and then began the treatment for consecutive three weeks, stop the drug for a week. The medicine was repeated for four cycles. Clean and disinfect the area before administration and conduct local anesthetic 30 minutes before operation. To use corresponding amount of solution according to different regions, varying from 1-10ml/100cm$^2$.

Subjects of the example: the 12 patients all met the standard of AS New York Classification revised in 1984 and their human leucocyte antigen were -B27 positive and in active period of AS with unilateral or bilateral lesions in hip joints. The subjects consisted of 8 male and 4 female AS patients aged from 18 to 40 years old (average age: 26 years old).

Mean index: the index of X ray of the hip joints:

Stage 0: normal, 0;
Stage I: suspicious change, local joint space may become narrower, 25 points;

Stage II: mildly abnormal, joint space becomes narrower obviously, the space > 2mm, 50 points;
Stage III: moderately abnormal, joint space becomes narrow, and the space < 2mm or the local joint space disappears, articular surfaces < 2mm ,75 points;
Stage IV: severe abnormal, bone deformation, articular surfaces > 2cm, 100 points.

If two of the three symptoms, namely, erosion and damage, syndesmophyte formation, arthrokatadysis appear during stage I and II, it should increase a level accordingly. BASRI evaluation was given according to different levels. Harris score: full score is 100 points, including pain degree of hip joints (44 points), function of hip joints(47 points), degree of lower limb malformation(4 points), range of activity of hip joints(5 points). Corresponding scores were given according to degrees. BASFI, BASDAI, ESR, CRP were applied in evaluating disease activity and function.

Results analysis of the example:

The activating agent of stem cells comprising chlorine dioxide is fully tolerant without local or systemic anaphylactic reaction. During the administration, the patients felt continuous pain and itchy which disappear completely after an hour.
As compared with the before administration, BASRI staging of the involvement hip joints was reduced half level three months after administration; BASRI staging of was reduced a level six months after administration.
As compared with the before treatment, the evaluation of Harris hip score, BASFI, BASDAI, ESP, CRP of the patients had a significant reduction. The differences were statistical significance ($P < 0.01$.) See Table 16 for the results.
Table 16 is the assessment of indexes before and after the treatment of AS

Table 16

| Items | Before treatment | the third month | the sixth month |
|---|---|---|---|
| BASRI Staging | 45±5 | 30±6* | 25±8* |
| Harris hip score | 82±6 | 49±5* | 32±4* |
| BASDAI | 6.4±1.8 | 3.6±1.5** | 3.0±1.2* |
| BASFI | 7.3±1.6 | 3.3±1.4** | 2.9±1.1* |
| ESR | 29.4±9.8 | 11.8±7.6** | 9.1±6.8** |
| CRP | 17.6±4.2 | 6.3±3.5** | 1.2±0.6** |
| *indicates P < 0.05, ** indicates P < 0.01 as compared with the before administration. | | | |

Ankylosing spondylitis(AS) is a systemic, immune and disability-causing disease characterized mainly by chronic inflammation of the axial joint. Lesions occur in peripheral articular and about 24%-75% of the patients, especially teenagers experience the lesions at the beginning or during the course of the disease. At present, there is no cure for AS, although treatments and medications can reduce symptoms and pain. Medications are used to treat the progression of the disease include hormone or biologicals. The activating agent of stem cells comprising a chlorine dioxide can solve the cause of the disease radically, and it can be promoted to treat other autoimmune diseases from the perspective of mechanism and experimental results, especially for various arthritis.
According to the example, the conclusion can be drawn by the skilled person in this field that the activating agent of stem cells in the present invention or the medicine made out of its main ingredients can be advantageously used in treating all kinds of autoimmune diseases and the mechanism of treatment is to promote tissue regeneration, regulate immune mechanism and repair injured tissue and organs by activating stem cells.

**Example 7**

The example is used to demonstrate the effects of activating agent of stem cells comprising chlorine dioxide on eczema as a medicine.
The subjects consisted of 12 patients with chronic eczema and average seven years of course of disease. External used medicines and oral administration, such as glucocorticoid and antihistamine drug were used discontinuously, but were poor in treatment. Activating agent was administered according to the following methods:

The first solution was a mixed solution made of sodium chlorite with the concentration of 8% and sodium chloride with the concentration of 2% with deionized water; the second solution was made of citric acid with the concentration of 12% with deionized water. Same volume of solutions were taken out from different vessels with different volumes (corresponding amount of solution were prepared according to different regions, varying from1-10ml/100cm$^2$.) and put into the suitable glass or plastic cups prepared. Allow the solution to mix for 3-5 minutes and then filter the solution with $0.22\mu$m filter membrane of double-deck. To smear solution over the affected parts immediately, once a day for consecutive 10 to 15 days, and then stop administration. If the eczema relapsed, reusing it again.

The 12 patients performed similar after treatment: After the administration, the itching feeling was replaced by stabbing pain, but both symptoms disappeared about half an hour. The affected parts scabbed after two to five days and disappeared after 10 to 15 days. The rash recovered to the normal thickness and the skin pigmentation lightened, clinical sign and pathological change of tissue became normal.

The 12 cases were traced for eight months and no recurrence happened for six of them; and four recurred once and did not recur after treatment for two times; two cases recurred twice and did not recur after the third treatment.

The functions of activating agent of stem cells comprising chlorine dioxide for eczema were the following: the feeling of stabbing pain can replace itching immediately; the oxidation of the medicine can eliminate the exogenous substance considered by the immune system; the effective ingredients of the medicine can activate the differentiation of the stem cells in the affected part, which can quicken wound healing and normalize the reaction for immune system; The oxidation of the medicine can protect the wound from infection by defensing against microoranism, such as bacterial.

According to the example, the conclusion can be drawn by the skilled person in this field that the activating agent of stem cells in the present invention or the medicine made out of its main ingredients can be advantageously used in treating all kinds of autoimmune skin diseases and the mechanism of treatment is to promote tissue regeneration, regulate immune mechanism and repair injured tissues and organs by activating stem cells.

**Example 8**

The example is used to demonstrate the effects of activating agent of stem cells comprising a chlorine dioxide on psoriasis.

Psoriasis is a T cell mediated inflammatory disease, one of the most commonly seen immune-mediated systemic diseases with 2%-3% of adult patients. The purpose of the example is to evaluate the effects of activating agent of stem cells comprising a chlorine dioxide on the immune-mediated systemic disease.

The subjects consisted of 86 patients with localized psoriasis aged from 28 to 68 years old, among which 43 patients were randomly assigned to treatment group, and 43 to control group. The disease broke out continuously with more than nine months of course of disease. The purpose of the clinical test was explained to all subjects and their consent was obtained before conducting the clinical test. Treatment group: 29 male patients(74.36%), 10 female(25.64%); age (49.05±14.57); course of disease (12±9) years. Control group: 28 male patients (68.29%), 13 female patients (31.71%); age (47.05±16.25); course of disease (12±10) years.

Administration method:

Administration of treatment group: The first solution was a mixed solution made of sodium chlorite with the concentration of 10% and sodium chloride with the concentration of 2.5% with deionized water; the second solution was made of citric acid with the concentration of 22% with deionized water. Same volume of solutions were taken out from different vessels (corresponding amount of solution were prepared according to different regions, varying from1-10ml/100cm$^2$.) and put into the suitable glass or plastic cups prepared. Allow the solution to mix for three to five minutes and then filter the solution with $0.22\mu$m filter membrane of double-deck. The solution was immediately smeared over the affected region, once a day for 10 to 15 days, and then stop administration. If it relapsed, reusing it again. Administration of control group: Triamcinolone acetonide and Econazole Nitrate Cream was smeared over the affected region, once a day for consecutive 10 to 15 days, and then stop administration. If it relapsed, reusing it again. The due time was six months.

The criteria for measuring the effect: Take PASI as reference.

A. The areas: grading from 0 to 6 for the four body parts respectively: area: 0= no rash; 1 < 10% ; 2=10 ~ 29% ; 3=30 ~ 49% ; 4=50 ~ 69% ; 4=50 ~ 69% ; 5=70 ~ 89%; 6=90 ~ 100% ; Trunk: head and face=10%; Trunk=30% ; upper limbs=20%; lower limbs=40%.

B. Severity of the clinic: erythema; infiltration; scale. Grading each characteristics from 0 to 4 point; 0=no ; 1=mild; 2=moderate; 3=severe; 4=extremely severe. Calculating the grades of each parts and working out the total points. The criteria of the effect (PASI decrease percentage): recovery score:≥95%; excellent score both≥61% and < 95% ; improved score both ≥20% and < 60%; No effect score < 20%.

Results analysis:

The example was enumeration data and a Chi-square test was conducted to compare the cure rate and recurrence rate of the two groups.

There were 43 patients in the treatment group, among which 41 were healed after at most three times of treatments, and the cure rate was 95.3%; Two patients were on the mend (See Table 17), 20 patients were treated more than twice, and nine patients were treated more than three times. There were 43 patients in control group, among which 30 patients were healed with at most three treatments. The cure rate was 69.8%. Ten patients were on the mend and three patients have curative effects, among which, 40 patients were treated more than two times, 38 patients were treated for three times. $P < 0.01$ as compared with the cure rates of two groups. Table 17 is the comparison of the effects between the the two groups after administration for 6 months.

Table 17

| Group | No. Patients | No. Cure and Percentage (%) | No.improvement and Percentage (%) | No. effective patients and percentage (%) | No.ineffective patients and percentage (%) |
|---|---|---|---|---|---|
| Treatment group | 43 | 41 (95.3%) | 2 (4.6%) | 0 (0) | 0 (0) |
| Control Group | 43 | 30 (69.8%) | 10 (23.3%) | 3(6.9%) | 0 (0) |

About recurrence (after 6 months)

35 of the 43 patients were conducted follow-up successfully in treatment group, and 32 of the 43 patients were interviewed successfully in control group (average time: 6 months), the recurrence rate of treatment group is 5.7% (2/35), the recurrence rate of control group is 71.9% (23/32) (See Table 18). $P < 0.01$ as compared with the two groups. Table 18 is about the recurrence.

Table 18

| Group | No. Patients | No. recurrence patients and percentage (%) | No. non recurrence patients and percentage (%) |
|---|---|---|---|
| Treatment group | 35 | 2 (5.7%) | 33 (94.3%) |
| Control group | 32 | 23 (71.9%) | 9 (28.1%) |

The functions of activating agent of stem cells comprising chlorine dioxide for psoriasis are the following: the feeling of stabbing pain can replace itching immediately; the oxidation of the medicine can eliminate the exogenous substance considered by the immune system; the effective ingredients of the medicine can activate the differentiation of the stem cells in the affected part, which can quicken wound healing and normalize the reaction for immune system; The oxidation of the medicine can protect the wound from infection by defensing against microorganism, such as bacterial.

According to the example, the conclusion can be drawn by the skilled person in this field that the activating agent of stem cells comprising a chlorine dioxide or the medicine made of its main ingredients can be advantageously used in treating all kinds of autoimmune disease and the mechanism of treatment is to promote tissue regeneration, regulate immune mechanism and repair injured tissue and organs by activating stem cells.

**Example 9**

The example is used to demonstrate the effects of activating agent of stem cells comprising a chlorine dioxide on rhinitis as a medicine.

Rhinitis is a common disease with high morbidity, and can be divided into chronic rhinitis and allergic rhinitis. There are few treatments for the lingering disease. The example aims at evaluating the effects of activating agent of stem cells comprising a chlorine dioxide on chronic and allergic rhinitis.

The subjects consisted of 30 patients with chronic and allergic rhinitis, and half of them were randomly assigned to treatment group and control group.

The administration for patients in treatment group:

The first solution was a mixed solution made of sodium chlorite with the concentration of 7.47% and sodium chloride with the concentration of 1.59% with deionized water; the second solution was made of citric acid with the concentration of 16.7% with deionized water. The solution comprising chlorine dioxide was made by mixing up the two solutions above. To make 50ml solution for each patient each time and put it into a bottle and stores it in the refrigerator. 2-5ml of solution for each patient each time was taken out and put into a beaker. The patients would breathe aiming at the cup-mouth for one to two minutes, and then the solution was diluted five to hundred times with normal saline before being put into nasal spray. To spray on each side of nose for three to five times. It was conducted once a day for consecutive 5 to 10 days. Then the administration was stopped, reuse the administration method for recurrence. The course of treatment was three months. The administration for patients in control group: use the ephedrine nose drops three times a day with three drops on each side each time for consecutive seven days. Reuse the administration method for recurrence. The course of treatment was three months.

Evaluation of the effects: The effects can be divided into excellent, effective and ineffective according to symptoms and signs. 1)Excellent: subjective symptoms disappear and positive sign become normal. 2)Effective: subjective symptoms and positive sign decrease obviously. 3) Ineffective: no significant effect was seen for subjective symptom and positive sign. Total effective rate(%)=(No.of excellent cases+No.of effective cases)/total cases×100%. Recurrence rate of rhinitis after treatment means the recurrence rate six months after treatment of all the cases. Table 19 is about the treatment of rhinitis.

Table 19

| Group | No. cases | Excellent | Effective | Ineffec tive | Effective rate | No. Recurrence | Recurrence rate |
|---|---|---|---|---|---|---|---|
| Treatment group | 30 | 26 | 3 | 1 | 97% | 3 | 10% |
| Control group | 30 | 10 | 13 | 7 | 77% | 27 | 90% |

As a medicine, the activating agent of stem cells comprising a chlorine dioxide has prominent effects on chronic and allergic rhinitis. It can not only contract nasal mucous obviously and decrease nasal gland secretion, but also has significant effect on reducing inflammation and allergy, which is superior to the frequently-used medicines.

The functions of activating agent of stem cells comprising chlorine dioxide for rhinitis are the followings: It is with a sense of stabbing pain and has antihistamine effect, which can reduce sneeze; the oxidation of the medicine can eliminate the exogenous substance considered by the immune system; the effective ingredients of the medicine can activate the differentiation of the stem cells in the affected part, which can quicken wound healing and normalize the reaction for immune system; The oxidation of the medicine can protect the wound from infection by defensing against microorganism, such as bacterial.

According to the example, the conclusion can be drawn by the skilled person in the field that the activating agent of stem cells comprising a chlorine dioxide or the medicine made of its main ingredients can be advantageously used to treat all kinds of inflammatory disease, anaphylactic disease, disease of respiratory system as well as ophthalmic diseases. The mechanism of treatment is to promote tissue regeneration, regulate immune mechanism and repair injured tissue and organs by activating stem cells.

**Example 10**

The example is used to demonstrate the effects of activating agent of stem cells comprising a chlorine dioxide on rat model of type II diabetes mellitus.

The first solution was a mixed solution made of sodium chlorite with the concentration of 4% and sodium chloride with the concentration of 1% with deionized water; the second solution was made of citric acid with the concentration of 8% with deionized water. Same volume of solutions were taken out from different vessels with different volumes and mixed up. Allow the solution to mix for 3-5 minutes and then filter the solution with $0.22\mu m$ filter membrane of double-deck, and put it into injection syringe immediately.

The rat models of type II diabetes mellitus bought from Department of Medicine of Peking University were divided into group A and B randomly with 10 rats for each group. Group A was treatment group of activating agent of stem cells. Group B was control group of 0.9% sodium chloride of the same volume. The rats of group A were injected with 0.5 ml activating agent of stem cells at insulin injection site on day 0, same dosage of medicine were injected on day 7, day 14; The rats of group B were injected with 0.5ml NS at hepatic artery at the same point-in-time, then calculate the blood sugar. The fasting blood-glucose of the rat model was checked on day 7, day 14, day 21 and day 28 respectively after the last injection. Table 20 is the results.

Table 20

| Group | MBG (mmol/L, ±SD) | | | | |
|---|---|---|---|---|---|
| | Day 0 Before administration | Day 7 after administration | Day 14 after administration | Day 21 after administration | Day 28 after administration |
| Group A | 17.3±0.4 | 9.3±3.5 | 4.3±1.1 | 3.5±1.3 | 3.1±1.2 |
| Group B | 16.5±0.7 | 18.3±2.4 | 17.2±1.6 | 16.7±1.9 | 17.9±2.3 |
| * indicates P < 0.01 as compared with the before administration using the Wilcoxon's test | | | | | |

The result indicates that the FBG levels of infected rats in treatment group become normal after 14 days, while that of control group remains unchanged. Therefore, activating agent of stem cells comprising a chlorine dioxide has prominent effect on rat model of type II diabetes mellitus, with few or no side effects.

According to the example, the conclusion can be drawn by the skilled person in the field that the activating agent of stem cells comprising a chlorine dioxide or the medicine made of it can be advantageously used in treating all kinds of diseases of urinary system and endocrine system disease. The mechanism of treatment is to promote tissue regeneration, repair injured tissue and organs by activating stem cells.

**Example 11**

The example is used to demonstrate the effects of activating agent of stem cells comprising a chlorine dioxide on gastrohelcosis as a medicine.

Gastrohecosis is a common and frequently-occurring disease of internal medicine with various causes. A major causative factor of gastrohelcosis is Helicobacter pylori infection. A great deal of research indicates that almost 80% of gastrohecosis results from HP infection. It is a typical disease resulting from injured gastric tissues due to the imbalance of the invasion and defense of mucosa.

The subjects consisted of 90 gastric ulcer patients (50 male and 40 female) of hp-positive aged from 33 to 75 years old (average age: 41 years old). The subjects were randomly assigned to treatment group and control group with 30 patients in treatment group and 30 in control group 1 and 30 in control group 2 respectively.

The first solution was a mixed solution made of sodium chlorite with the concentration of 20% and sodium chloride with the concentration of 5% with deionized water; the second solution was made of citric acid with the concentration of 30% with deionized water. Same volume of solutions were taken out from different vessels with different volumes and put into the suitable glass or plastic cups prepared. Allow the solution to mix for 3-5 minutes. The capsule that containing activating agent of stem cells was made by injecting the mixed solution into the soft capsule of number 0.

The capsule that containing activating agent of stem cells was administered to the patients in treatment group three times a day and a pill each time; 30 minutes before meals, patients in control group 1 took orally 15mg colloidal bismuth and 100mg pectin tinidazole for three times a day. And 20mg Omeprazole two times a day; patients in control group 2 took orally 1.0mg amoxicillin and 500mg clindamycin three times a day and 20mg esomeprazole twice a day before meals. Both groups were administered for a consecutive week. To observe the improvement of the clinical symptoms of the three cases two weeks after administration, such as stomachache, nausea and sour regurgitation. Gastroscopy and urease test of gastric antral mucosa were conducted after 30 days to observe ulcer healing and eradication of hp.

Criteria for measuring the effects: the changes of conditions before and after treatment and the time the symptoms disappear were recorded precisely. Diagnostic criteria are the followings: 1)complete healing: the symptoms disappear, such as ache, heartburn, nausea, sour regurgitation, abdominal distention; cure of ulceration under a gastroscope and the inflammation is gone; H pylori is negative. 2) Almost healing: subjective symptom disappear, and more than 90% ulceration are healed and the inflammation around is gone. 3)Effective: more than half of the ulcer surface shrink or more than half of ulceration reduced; 4)Ineffective: less than half of the ulceration are healed or haven't been changed, or even the ulcer surface or ulceration increase. To conduct rapid urease test by taking one sample of gastric mucosa at antrum of stomach and gastric body respectively, if the results are negative, it means Hp negative conversing.

See Table 21 for the results.

Table 21

| Group | Symptoms disappear after a week | Complete healing after 30d | Healing after 30d | Effective after 30d | HP negative conversing |
|---|---|---|---|---|---|
| Treatment group | 30 (100%) | 28 (93.3%) | 1 (3.3%) | 1 (3.3%) | 29 (96.6%) |
| Control group 1 | 30 (100%) | 24 (80%) | 3 (10%) | 3 (10%) | 29 (96.6%) |
| Control group 2 | 26 (86.7%) | 20 (66.7%) | 5 (16.7%) | 5 (16.7%) | 18 (60%) |

It can be seen that the HP negative conversing rate of treatment group was comparative to that of control group 1, which indicated that the activating stem cells of the present invention fully played its role in killing the pathogentic microorganism; Although the effect of eradicating HP was almost the same, the complete healing rate of gastric ulcer with the medicine made by activating stem cells is higher. Thus, the activating agent of stem cells in the invention promoted the regeneration of tissue and quickened healing.

According to the example, the conclusion can be drawn by the skilled person in this field that the activating agent of stem cells comprising a chlorine dioxide or the medicine made of it can be advantageously used in treating all kinds of stomach diseases and the disease caused by pathogenic microorganism of organ tissues(except for skin), the mechanism of treatment is to promote tissue regeneration, repair injured tissue and organs by activating stem cells, and kill pathogentic microorganism by chlorine dioxide.

## Example 12

The example is to observe the preventive effects of activating agent of stem cells comprising a chlorine dioxide on postoperative complications of lung cancer.

Operative treatment is the first choice for lung cancer, but it can cause a series of postoperative complications easily because of the severe traumatic. The followings are the common complications: 1)anastomotic fistula in bronchus and bronchopleural fistula; 2) Post-operative pleural hemorrhage; 3)Respiratory complications; 4) The cardiovascular system complications.

The subjects consisted of 30 lung cancer patients of stage I, II III who had operations on lung cancer just now. The subjects were randomly assigned to treatment group and control group with 15 patients respectively.

15 ml acid aqueous solution with a ph value of 3 was made by citric acid and deionized water. 500 ml aqueous solution of 30% was made with deionized water and sodium chlorite, 500ml aqueous solution of 50% was made with deionized water and citric acid. To mix the above solutions in a conical beaker of 2L capacity and then put them in the prepared acid aqueous solution by a plastic tube with bottle stopper. The activating agent of stem cells comprising chlorine dioxide was made by bubbling chlorine dioxide in the acid aqueous solution for 40 minutes and the solution was contained in 150 plastic bottles with 100ml capacity and stored in the fridge.

Each patient in treatment group received 10 bottles of the solution. Open the plastic bottle cap and do alternate nostril breathing for 2-10 minutes on day 2 after the operation with a bottle a day for three times, then cover the bottleneck once finished. No special nursing means were applied in control group, then observe the complications a month after the operation.

Postoperative complications of lung cancer can be divided into three levels: Serious complications: need to see the doctor; minor complications: the patients can deal with by themselves; No complication: none was observed in clinic.

Table 22 is about the postoperative complications of lung cancer of 30 patients

Table 22

| Group | Serious complications | Minor complications | No complication |
|---|---|---|---|
| Treatment group | 1 (6.7%) | 5 (33.3%) | 9 (60%) |
| Control group | 4 (26.7%) | 6 (40%) | 5 (33.3%) |

From Table 22, the conclusion can be drawn that activating agent of stem cells comprising a chlorine dioxide has significant preventive effects on postoperative complications of lung cancer. It indicates that the activating agent can promote injured tissue regeneration caused by cancer, and repair the normal function of tissues, and it can be regarded as a supplementary means for cancer.

According to the example, the conclusion can be drawn by the skilled person in the field that the activating agent of stem cells comprising a chlorine dioxide or the medicine made of it can be advantageously used in promoting the regeneration of tissue of various cancer and treating disease of respiratory system. Besides, the drug action mechanism is to promote tissue regeneration and repair injured tissue and organs by activating stem cells.

**Example 13**

The example is used to demonstrate the effects of activating agent of stem cells comprising a chlorine dioxide on removing scar.

Scars are areas of fibrous tissue (fibrosis) that replace normal skin after injury. A scar results from the physical, biological, chemical process of wound repair in the skin and other tissues of the body. Actually, scar is an abnormal and diseased tissue that is not provided with normal tissue structure of skin and physiological function. In theory, the scar can be removed or treated by debridement, that is, to stimulate the regeneration of stem cells of normal tissues, so that the scar tissues could be replaced by normal tissues.

The subjects consisted of 20 patients with scar, including 7 male and 13 female(average age:33 years old.) There are five cases with burn scar, 7 cases with scars resulting from accidental injury, 5 cases with surgical scar, 3 cases with scar from skin disease. The average course of disease is about 3 years.

The first solution was a mixed solution made of sodium chlorite with the concentration of 10% and sodium chloride with the concentration of 2.5% with deionized water; the second solution was made of citric acid with the concentration of 20% with deionized water. Same volume of solutions were taken out from different vessels(corresponding volume of solution were prepared according to different regions, varying from 1-10ml/100cm$^2$.) and put into the suitable glass or plastic cups prepared. Allow the solution to mix for 3-5 minutes and then filter the solution with 0.22$\mu$m filter membrane of double-deck, dimethyl sulfoxide was added in the rate of 1:1 of the solution. Then the solution was immediately smeared over the scar, once a day for 10-15 days consecutively then stops administration. The second administration began 1-2 months after drug withdrawal of the first administration, once a day for consecutive 5-10 days.

To observe the effects a month after first administration and second administration. The effects were measured according to pain, stiffness and hyperpigmentation around.

1) specific criteria: obvious pain (+++), moderate pain (++), mild pain (+) no pain (-). stiffness of scar: can't sunken with hard pressing, (+++) can sunken with moderate pressing, (++), complete depression with mild pressing (+) .Same character with normal skin (-) .hyperpigmentation around : >2cm, (+++), 1 ~ 2cm (++), <1cm (+), 0 (-)

2) Measurement of clinical effect: excellent: no pain, and the stiffness is the same to normal skin with no hyperpigmentation around. effective: the indicators of pain, stiffness and hyperpigmentation are (+) ~ (++); ineffective: one of the indicators of pain, stiffness and hyperpigmentation is (+++).

Table 23 is the effects of the 20 patients with scar

Table 23

| a month after first administration | | | a month after second administration | | |
|---|---|---|---|---|---|
| Efficiency rate | total efficiency | Ineffective | Efficiency rate | total efficiency | ineffective |
| 9 (45%) | 6 (30%) | 5 (25%) | 15 (75%) | 5 (25%) | 0 |

A month after the first administration, the effective rate of scar treatment was 45% and the total efficiency was 30% with the medicine made of the activating agent of stem cells. Efficiency rate increased to 75% a month after the second administration. It can be seen that the medicine had a significant effect on skin scar. It can be found that the activating agent of stem cells first removed the scar tissue by its unique debrideme, and then activated stem cells for regeneration, so as to grow complete skin with new function. Therefore, the activating agent of stem cells containing a chlorine dioxide can be applied in regeneration of tissue in aesthetic and plastic surgery.

According to the example, the conclusion can be drawn by the skilled person in this field that the activating agent of stem cells comprising a chlorine dioxide or the medicine made of it can be advantageously used in tissue regeneration in terms of plastic and aesthetic surgery, and also treating general skin disease. Besides, the mechanism of treatment is to promote tissue regeneration and repair injured tissues and organs by activating stem cells.

## Claims

1. A stem cells activating agent comprising chlorine dioxide for use for the treatment of an autoimmune disease, wherein the stem cells activating agent activates proliferation, migration and differentiation of stem cells in the targeted tissue of an animal.

2. A stem cells activating agent for use according to claim 1, wherein the stem cells activating agent containing chlorine dioxide is prepared as follows: dissolving chlorine dioxide gas in an acidic solution A with pH value of 1.5 ~ 6.5 containing an acidic pH adjuster, so as to prepare chlorine dioxide solution of 500 ~ 2900 ppm.

3. A stem cells activating agent for use according to claim 1, wherein the stem cells activating agent containing chlorine dioxide is prepared as follows: dissolving precursor of chlorine dioxide in water to make an aqueous solution with concentration of 1% ~ 40%, and then adding an acidic solution B containing an acidic pH adjuster to the aqueous solution so as to adjust pH value of the resultant mixed solution to 1.5 ~ 6.5.

4. A stem cells activating agent for use according to claim 1, wherein the stem cells activating agent containing chlorine dioxide is prepared as follows: dissolving precursor of chlorine dioxide in water to make an aqueous solution C with concentration of 1% ~ 40%, and dissolving an acidic pH adjuster in water to make an acidic solution D, and mixing the solution C with the solution D to adjust the pH value of the resultant mixed solution to 1.5 ~ 6.5 before using.

5. A stem cells activating agent for use according to claim 3 or 4,, wherein the precursors of chlorine dioxide is sodium chlorite, potassium chlorite, lithium chlorit, calcium chlorite, magnesium chlorate or barium chlorite.

6. A stem cells activating agent for use according to claim 2, 3 or 4 wherein the acidic pH adjusters is citric acid, acetic acid or monosodium phosphate.

7. A stem cells activating agent for use according to claim 1, wherein the autoimmune diseases include thrombocytopenic purpura, leukopenia, pemphigus, pemphigoid, Graves' disease, myasthenia gravis, urticarial vasculitis, systemic lupus erythematosus, rheumatoid arthritis, polyarteritis, contact dermatitis,
discoid lupus erythematosus, angiitis, type I diabetes, multiple sclerosis, psoriasis, psoriatic arthritis, scleroderma, encephalomyelitis, alopecia areata,
amyotrophic lateralizing sclerosis, ankylosing spondylitis, autoimmune urticaria, bullous pemphigoid, chronic obstructive pulmonary disease, cicatricial pemphigoid, herpetic dermatitis, dermatomyositis, diffuse systemic sclerosis, eczema, anaphylactoid purpura, idiopathic inflammatory demyelinating diseases, neurodermatitis, lichen sclerosis, amyotrophic lateralizing sclerosis, scleroderma, palindromic rheumatism, polyarteritis nodose, polymyalgia rheumatica, sicca syndrome and leucoderma.

## Patentansprüche

1. Ein trockener Zellaktivator, es enthält Chlordioxid zur Behandlung von Autoimmunerkrankungen, wobei: Der Stammzellenaktivator aktiviert die Proliferation, Migration und Differenzierung von Stammzellen im Zielgewebe des Tieres.

2. Ein Stammzellenaktivierungsmittel zur Verwendung gemäß Patentanspruch 1, **dadurch gekennzeichnet, dass**: Das Herstellungsverfahren des Chlordioxids enthaltenden Stammzellenaktivierungsmittels ist wie folgt: Chlordioxidgas wird in einer Lösung A gelöst, die einen pH-Wert zwischen 1,5 und 6,5 aufweist und ein saures pH-Einstellmittel enthält. Herstellung einer Konzentration von 500 ~ 2900 ppm Chlordioxidlösung.

3. Ein Stammzellenaktivierungsmittel zur Verwendung gemäß Patentanspruch 1, **dadurch gekennzeichnet, dass**: Das Herstellungsverfahren des Chlordioxids enthaltenden Stammzellenaktivierungsmittels ist wie folgt: Der Chlordioxidvorläufer wird in Wasser gelöst, um eine Konzentration von 1% bis 40% wässeriger Lösung zu erhalten, dann wird eine saure Lösung B, die ein saures pH-Einstellmittel enthält, zu der wässrigen Lösung gegeben. Der pH-Wert der erhaltenen gemischten Lösung wurde auf 1,5 bis 6,5 eingestellt.

4. Ein Stammzellenaktivierungsmittel zur Verwendung gemäß Patentanspruch 1, **dadurch gekennzeichnet, dass**: Das Herstellungsverfahren des Chlordioxids enthaltenden Stammzellenaktivierungsmittels ist wie folgt: Der Chlordioxidvorläufer wird in Wasser gelöst, um eine Konzentration von 1% bis 40% wässeriger Lösung C zu erhalten, dann Auflösen eines sauren pH-Einstellmittels in Wasser, um eine saure Lösung D zu erhalten, und Mischen Sie der Lösung C und der Lösung D. Und stellen Sie den pH-Wert der resultierenden Mischlösung vor Gebrauch auf 1,5 bis 6,5 ein.

**5.** Ein Stammzellenaktivierungsmittel zur Verwendung gemäß Patentanspruch 3 oder 4, **dadurch gekennzeichnet, dass**: Die Chlordioxidvorläufer sind Natriumchlorit, Kaliumchlorit, Lithiumchlorit, Kaliumchlorit, Natriumchlorid, Kaliumchlorid, Magnesiumchlorat oder Bariumchlorit.

**6.** Ein Stammzellenaktivierungsmittel zur Verwendung gemäß Patentanspruch 2, 3 oder 4, **dadurch gekennzeichnet, dass**: Bei dem sauren pH-Einstellungsmittel handelt es sich um Zitronensäure, Essigsäure oder Natriumdihydrogenphosphat.

**7.** Ein Stammzellenaktivierungsmittel zur Verwendung gemäß Patentanspruch 1, **dadurch gekennzeichnet, dass**: Die Autoimmunkrankheiten umfassen:

thrombozytopenische Purpura, Leukopenie, Pemphigus, Pemphigoid, Morbus Basedow, Myasthenia Gravis, Urtikariavaskulitis, systemischer Lupus erythematodes, rheumatoide Arthritis, Polyarteritis, Kontaktdermatitis, Discoider Lupus erythematodes, Vaskulitis, Diabetes mellitus Typ I, Multiple Sklerose, Psoriasis, Psoriasis-Arthritis, Enzephalomyelitis, Sklerodermie, Alopecia areata, Amyotrophe Lateralsklerose, Spondylitis ankylosans, Autoimmunurtikaria, bullöses Pemphigoid, chronisch obstruktive Lungenerkrankung, Vernarbungen Pemphigoid, Dermatitis herpetiformis, Dermatomyositis, Lichen Sclerosis, diffuse systemische Sklerose, Neurodermitis, Allergische Purpura, idiopathische entzündliche demyelinisierende Krankheit, Neurodermitis, amyotrophe Lateralsklerose, Sklerodermie, Palindromischer Rheumatismus, Polyarteritis nodosa, Polymyalgia rheumatica, Sjögren-Syndrom und Vitiligo.

## Revendications

**1.** Un agent activateur de cellules souches comprenant du dioxyde de chlore à utiliser pour le traitement d'une maladie auto-immune, dans lequel l'agent activateur de cellules souches active la prolifération, la migration et la différenciation de cellules souches dans le tissu ciblé d'un animal.

**2.** Un agent activateur de cellules souches destiné à être utilisé selon la déclaration 1, dans lequel l'agent d'activation de cellules souches contenant du dioxyde de chlore est préparé comme suit: dissoudre du gaz de dioxyde de chlore dans une solution acide A ayant un pH compris entre 1,5 et 6,5, contenant un ajusteur de PH, de manière à préparer une solution de dioxyde de chlore de 500 - 2900 ppm.

**3.** Un agent activateur de cellules souches utilisable selon la revendication 1, dans lequel l'agent d'activation de cellules souches contenant du dioxyde de chlore est préparé comme suit: dissoudre le précurseur de dioxyde de chlore dans l'eau pour obtenir une solution aqueuse à 1% -40% puis ajouter une solution acide B contenant un ajusteur acide de pH à la solution aqueuse de manière à ajuster le pH de la solution mélangée résultante entre 1,5 et 6,5.

**4.** Un agent activateur de cellules souches utilisable selon la déclaration 1, dans lequel l'agent activateur de cellules souches contenant du dioxyde de chlore est préparé comme suit : dissoudre le précurseur de dioxyde de chlore dans l'eau pour obtenir une solution aqueuse C à 1% -40%, et dissoudre un ajusteur acide de pH dans l'eau pour préparer une solution acide D et mélanger la solution C avec la solution D pour ajuster la valeur du pH de la solution mélangée résultante, à 1,5-6,5 avant utilisation.

**5.** Un agent activateur de cellules souches utilisable selon la revendication 3 ou 4, dans lequel les précurseurs du dioxyde de chlore sont le chlorite de sodium, le chlorite de potassium, le chlorite de lithium, le chlorite de calcium, le chlorate de magnésium ou le chlorite de baryum.

**6.** Un agent activateur de cellules souches à utiliser selon la revendication 2, 3 ou 4, dans lequel les ajusteurs de pH acides sont l'acide citrique, l'acide acétique ou le phosphate monosodique.

**7.** Un agent activateur de cellules souches utilisable selon la revendication 1, dans lequel les maladies auto-immunes suivantes : le purpura thrombocytopénique, la leucopénie, le pemphigus, la pemphigoïde, la maladie de Graves, la myasthénie, la vascularite urticarienne, le lupus érythémateux disséminé, la polyarthrite rhumatoïde, la dermatite de contact, cystite interstitielle, angiite, diabète de type I, sclérose en plaques, psoriasis, rhumatisme psoriasique, sclérodermie, encéphalomyélite, alopécie, sclérose latérale amyotrophique, spondylarthrite ankylosante, urticaire auto-immune, pemphigoïde bulleuse, maladie pulmonaire obstructive chronique, pemphigoïde cicatricielle, dermatite herpétique, dermatomyosite , sclérose systémique diffuse, eczéma, purpura anaphylactoïde, maladies démyélinisantes inflammatoires idiopathiques, neurodermatite, sclérose du lichen, sclérose amyotrophique latérale, sclérodermie, rhumatisme palindromique, polyarthrite nodulaire (nodules d'arthrite rhumatoïde), polymyalgie rhumatismale, syndrome d'insensibilité complète aux androgènes (SICA) et vitiligo.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101405021 A
- CN 101278045 A
- CN 101657206 A
- CN 102137651 A
- CN 101641104 A
- CN 1199633 C
- US 5750108 A
- CN 102441006 A
- WO 8910747 A1
- WO 2011090932 A2
- WO 2010111137 A2

**Non-patent literature cited in the description**

- *Science,* 2000, vol. 287, 1442-1446
- *Tissue Eng,* 2005, vol. 11, 497-505
- *Transplantation Proceedings,* 2005, vol. 37, 273-275
- **GARRY D J et al.** Ponce de Leon's fountain: stem cells and the regenerating heart. *Am J Med Sci,* 2005, vol. 329 (4), 190-201
- **WEISSMAN I L.** Translating stem and progenitor cell biology to the clinic: Barriers and opportunities. *Science,* 2000, vol. 287, 1442-1446
- **GARRY D J et al.** Ponce de leon's fountain: stem cells and the regenerating heart. *Am J Med Sci,* 2005, vol. 329 (4), 190-201
- **REYNOLDS B A ; WEISS S.** Generation of neurons and astrocytes from isolated cells of the adult mammalian central nervous system. *Science,* 1992, vol. 255, 1707-1710
- **SHIHABUDDIN L S et al.** Adult spinal cord stem cells generate neurons after transplantation in the adult dentate gyrus. *The J Neuroscience,* 2000, vol. 20, 8727-8735
- **SHEN Q et al.** Endothehal cells stimulate self-renewal and expand neurogenesis of neural stem cells. *Science,* 2004, vol. 304, 1338-1440
- **YAOJIONG WU et al.** Mesenchymal Stem Cells Enhance Wound Healing Through Differentiation and Angiogenesis. *STEM CELLS,* October 2007, vol. 25 (10), 2648-2659
- **TERAMOTO T et al.** EGF amplifies the replacement of parvalbumin-expressing striatal interneurons after ischemia. *The J Clinical Investigation,* 2003, vol. 111, 1125-1132
- **WALTER D H et al.** Statin therapy accelerates reendothelialization: A novel effect involving mobilization and incorporation of bone marrow-derived endothelial progenitor cells. *Circulation,* 2002, vol. 105, 3017-3024
- **TAKEYAMA K.** Ohto H: PBSC mobilization. *Transfus Apher Sci,* 2004, vol. 31, 233-243
- **AICHER A ; ZEIHER A M ; DIMMELER S.** Mobilizing endothelial progenitor cells. *Hypertension,* 2005, vol. 45, 321-325
- **LORD ; WRIGHT.** *Blood Cells,* 1980, vol. 6, 581-593
- **WRIGHT ; LORIMORE.** *Cell Tissue Kinet,* 1987, vol. 20, 191-203
- **MARSHAL LAND LORD.** *Int Rev. Cyt.,* 1996, vol. 167, 185-261
- **MARRACK et al.** *Nat Med,* 2001, vol. 7, 899-905
- **VIKASH CHANDRA et al.** Islet-Like Cell Aggregates Generated from Human Adipose Tissue Derived Stem Cells Ameliorate Experimental Diabetes in Mice. *PLoS One,* 2011, vol. 6 (6), e20615
- **WEI CAO et al.** Leukemia Inhibitory Factor Inhibits T Helper 17 Cell Differentiation and Confers Treatment Effects of Neural Progenitor Cell Therapy in Autoimmune Disease. *Immunity,* 11 August 2011, vol. 35 (2), 273-284
- **CHUTIMA TALCHAI et al.** Generation of functional insulin-producing cells in the gut by Foxo1 ablation. *Nature Genetics.,* 2012, vol. 44, 406-412
- **YONG ZHAO ; ZHAOSHUN JIANG ; TINGBAO ZHAO ; MINGLIANG YE ; CHENGJIN HU ; ZHAOHUI YIN et al.** Reversal of type 1 diabetes via islet beta cell regeneration following immune modulation by cord blood-derived multipotent stem cells. *BMC Medicine 2012,* 10 January 2012, vol. 10, 3
- **YU, J et al.** Intravenous Administration of Bone Marrow Mesenchymal Stem Cells Benefits Experimental Autoimmune Myasthenia Gravis Mice Through an Immunomodulatory Action. *Scandinavian Journal of Immunology,* 2010, vol. 72 (3), 242
- **JULIA M. RUCKH et al.** Rejuvenation of Regeneration in the Aging Central Nervous System. *Cell Stem Cell,* 06 January 2012, vol. 10 (1), 96-103

- **KENTARO AKIYAMA et al.** Mesenchymal-Stem-Cell-Induced Immunoregulation Involves FAS-Ligand-/FAS-Mediated T Cell Apoptosis. *Cell Stem Cell,* 26 April 2012, vol. 10 (5), 544-444
- **PITTENGER, M.F. ; MARTIN, B.J.** Mesenchymal stem cells and their potential as cardiac therapeutics. *Circ. Res.,* 2004, vol. 95, 9-20
- **JIN WEI et al.** the trial study on promotion of chlorine dioxide on wound healing of rabbit. *Shanxi Medical University Journal,* vol. 42 (7), 2011
- **KATHERINE LAU et al.** xploring the role of stem cells in cutaneous wound healing. *Experimental Dermatology,* November 2009, vol. 18 (11), 921-933
- **CLIFFORD J. WOOLF.** What is this thing called pain. *J Clin Invest.,* 01 November 2010, vol. 120 (11), 3742-3744
- **CLIFFORD J. WOOL.** Pain: Moving from Symptom Control toward Mechanism-Specific Pharmacologic Management. *Annals of Internal Medicine,* 2004, vol. 140 (6), 441-451
- **KATHERINE LAU et al.** Exploring the role of stem cells in cutaneous wound healing. *Experimental Dermatology,* November 2009, vol. 18 (11), 921-933
- **ITO M et al.** Wnt-dependent de novo hair follicle regeneration in adult mouse skin after wounding. *Nature,* 17 May 2007, vol. 447 (7142), 316-20
- **HILA TOLEDANO et al.** The let-7-Imp axis regulates ageing of the Drosophila testis stem-cell niche. *Nature,* 2012
- **MAJIDA RIZK et al.** Nitric Oxide and Wound Healing. *World Journal of Surgery,* 2004, vol. 28 (3), 301-306
- **M. B. WITTE et al.** Nitric oxide enhances experimental wound healing in diabetes. *British Journal of Surgery,* December 2002, vol. 89 (12), 1594-1601
- **ALLON M. KLEI et al.** Universal patterns of stem cell fate in cycling adult tissues. *Development,* 01 August 2011, vol. 138, 3103-3111
- **WEISSMAN IL.** Translating stem and progenitor cell bilolgy to the clinic: Barriers and opportunities. *Science,* 2000, vol. 287, 1442-1446
- **GARRY DJ et al.** Ponce de Leon's fountain: stem cells and the regenerating heart. *Am J Med Sci.,* 2005, vol. 329 (4), 190-201
- Stem Cell Antholgy. Elsevier Inc, 2010
- **LUIS A. GARZA et al.** Bald scalp in men with androgenetic alopecia retains hair follicle stem cells but lacks CD200-rich and CD34-positive hair follicle progenitor cells. *J Clin Invest.,* 01 February 2011, vol. 121 (2), 613-622
- **ITO M ; LIU Y ; YANG Z ; NGUYEN J ; LIANG F ; MORRIS RJ et al.** Stem cells in the hair follicle bulge contributed to wound healing, but not to homeostasis of the epidermis. *Nat Med.,* 2005, vol. 11, 1351-4
- **ABIGAIL K LANGTON et al.** An Extended Epidermal Response Heals Cutaneous Wounds in the Absence of a Hair Follicle Stem Cell Contribution. *Journal of Investigative Dermatology,* 2008, vol. 128, 1311-1318
- **ZHAO Y et al.** A human peripheral blood monocyte-derived subset acts as pleuripotent stem cells. *Proc Natl Acad Sci USA,* 2003, vol. 100, 2426-31
- **MICHEJDA M.** Which stem cells should be used for transplantation. *Fetal Diagn Ther,* 2004, vol. 19, 2-8
- **FADINI G P et al.** Circulating endothelial progenitor cells are reduced in peripheral vascular complications of tape 2 diabetes mellitus. *J American College of Cardiology,* 2005, vol. 45, 1449-1457
- **KUWANA M et al.** Human circulating CD14+ monocytes as a source of progenitors that exhibit mesenchymal cell differentiation. *J Leukoc Biol,* 2003, vol. 74, 833-845
- **ASAHARA T et al.** Isolation of putative progenitor endothelial cells for angiogenesis. *Science,* 1997, vol. 275, 964-967
- **HRISTOV M.** Weber C. Endothelial progenitor cells: characterization, pathophysiology, and possible clinical relevance. *J Cell Mol Med,* 2004, vol. 8, 498-508
- **HRISTOV M ; WEBER C.** Endothelial progenitor cells: characterization, pathophysiology, and possible clinical relevance. *J Cell Mol Med,* 2004, vol. 8, 498-508
- **LONGA E Z et al.** Reversible middle cerebral artery occlusion without craniectomy in rats. *Stroke,* 1989, vol. 20, 84-91